# EUROPEAN PATENT SPECIFICATION

(11) **EP 4 081 264 B1**
(45) Date of publication and mention of the grant of the patent: **21.08.2024**
(21) Application number: 20845815.8
(22) Date of filing: 22.12.2020
(51) Int. Cl.: A61L 2/10, A61L 2/22, A61L 2/24, B05B 16/80, B05B 16/00, H01L 33/18, G06K 19/077, G08B 21/24, G16H 40/20, G16H 40/67

(54) **MODULAR APPARATUS FOR DISINFECTING OBJECTS INCLUDING SENSOR SYSTEMS AND TRACKING MECHANISMS**
APPAREIL MODULAIRE DE DÉSINFECTION D'OBJETS COMPRENANT DES SYSTÈMES DE CAPTEURS ET DES MÉCANISMES DE SUIVI
MODULARE VORRICHTUNG ZUR DESINFEKTION VON OBJEKTEN MIT SENSORSYSTEMEN UND NACHVERFOLGUNGSMERKMALEN

(30) Priority: 24.12.2019 US 201962953373 P
(43) Date of publication of application: 02.11.2022
(73) Proprietor: UV-Concepts, Inc., Littleton, CO 80120 (US)
(72) Inventor: STARKWEATHER, Jeremy, Castle Rock, Colorado 80108 (US); YLIZARDE, Jason, Conroe, Texas 77384 (US); WYNNE, John, Cincinnati, Ohio 45242 (US); EDMUNDOWICZ, Brent, Centennial, Colorado 80016 (US); STARKWEATHER, Austin, Denver, Colorado 80237 (US); WENGER, Stefan L., Columbine Valley, Colorado 80123 (US)
(74) Representative: Cooley (UK) LLP
(86) International application number: PCT/US2020/066644
(87) International publication number: WO 2021/133815

(56) References cited:
- WO-A1-2019/246394
- US-A1- 2015 205 985
- US-A1- 2019 365 938

## Description

### CROSS-REFERENCE TO RELATED APPLICATIONS

This application claims priority to U.S. Provisional Patent Application No. 62/953,373, filed December 24, 2019, titled "Modular Components, Systems, and Methods for Disinfecting Objects".

### TECHNICAL FIELD

The present invention relates generally to a modular apparatus for disinfecting objects. More specifically, the present invention relates to disinfecting structures formed of modular units that include energy sources, such as light sources capable of emitting ultraviolet (UV) light, which can be used to disinfect objects, including equipment within a medical facility.

### BACKGROUND

Disinfection of objects and spaces can reduce the transmission of pathogens. In medical facilities, disinfection of equipment, instruments, and other objects is important to prevent the spread of illnesses between individuals. Disinfection can be accomplished using, for example, UV light or other energy sources and/or disinfecting agents.

The effectiveness of a disinfection system can depend on the physical setting and/or method of disinfection. For example, with UV disinfection, it has been shown that intensity, proximity, and line of sight affect the ability of UV light emitted from a disinfection system to effectively eliminate pathogens on equipment and/or within spaces. But many existing UV disinfection systems, once installed within a medical setting, are stationary. For example, UV disinfection stations for disinfecting publicly-used equipment are described by Taylor et al. in U.S. Patent Nos. 7,791,044 and 8,536,541. The stationary units described in these patents are particularly useful for disinfecting mobile equipment, such as shopping carts, wheelchairs, gurneys, etc. Because the units are stationary, however, the units may have limited applications, e.g., be designed for a specific space and/or type of equipment. When changes occur with the space and/or equipment, the disinfection units may need to be manually moved, adapted, or replaced.

In addition, existing systems capable of disinfecting larger scale objects and/or spaces may have dimensions that make them difficult to transport and deploy onsite. For example, such systems may have dimensions larger than standard size doorways and/or openings within a medical facility, and therefore require onsite construction and/or disassembly and reassembly to get through doorways and/or openings. Once the disinfection systems are assembled within a room or area, the systems may have limited mobility and/or adaptability. For example, such systems may be formed of a single, unitary structure that requires the entire system to be replaced (or a large portion of the system to be replaced) when individual, smaller components fail or require replacement over time. Such systems may also be difficult to move due to their large size and/or weight, be difficult to modify based on changes to equipment being disinfected and/or changes to the onsite location of the system, etc. These limitations and others can lead to significant costs, including downtime costs when a system is being installed, repaired, modified, and/or moved, and associated labor costs.

US2019/365938 discloses a sterilization unit which includes: a UV-C radiation source configured to emit UV-C radiation; and a room partition selectably configurable between two or more different partition geometries and configured, in each of the two or more different partition geometries, to (a) physically separate floor space of a room into sterilization target area and a non-target area, and (b) direct the UV-C radiation to the target area from at least two different directions while shielding the non-target area from the UV-C radiation.

US2015/205985 discloses a method for organizing the disinfection of one or more items contaminated with biological agent(s) comprising (a) attaching a radio-frequency ID (RFID) tag to an item(s) to be disinfected; exposing the item(s) of (a) to a disinfecting means for a period sufficient to disinfect the item; and (c) obtaining a signal from the tagged item when disinfection is complete thereby organizing the disinfection of one or more items.

EP3787697 (Art. 54(3) EPC) discloses a disinfecting system formed of a plurality of modular units, wherein each modular unit is coupleable to at least one other modular unit from the plurality of modular units and includes an energy source from a plurality of energy sources. The plurality of energy sources can be configured to provide energy having an intensity capable of disinfecting a surface of the object located in a disinfecting area.

### SUMMARY

The apparatus described herein can overcome some of the disadvantages associated with existing disinfection systems. In particular, the apparatus described herein relate to disinfection systems having modular components.

According to the invention, an apparatus includes the features of claim 1.

In some embodiments, the apparatus includes the features of the dependent claims.

### BRIEF DESCRIPTION OF THE DRAWINGS

The skilled artisan will understand that the drawings primarily are for illustrative purposes and are not intended to limit the scope of the inventive subject matter described herein. The drawings are not necessarily to scale; in some instances, various aspects of the inventive subject matter disclosed herein may be shown exaggerated or enlarged in the drawings to facilitate an understanding of different features. In the drawings, like reference characters generally refer to like features (e.g., functionally similar and/or structurally similar elements).
FIG. 1 schematically illustrates an example of a disinfection system, according to embodiments disclosed herein.
FIG. 2A schematically illustrates an example of a disinfection system including modular units, according to embodiments disclosed herein.
FIG. 2B schematically illustrates an example of a modular unit of a disinfection system, according to embodiments disclosed herein.
FIGS. 3A-3C schematically illustrate different configurations of modular units of disinfection systems, according to embodiments disclosed herein.
FIG. 4 schematically illustrates an example of a side panel of a disinfection system formed of modular units, according to embodiments disclosed herein.
FIGS. 5A, 5B, 5C, and 5D schematically illustrate different views of an example disinfection system including modular units, according to embodiments disclosed herein. FIGS. 5A and 5B depict a top view of the disinfection system, with a movable panel of the disinfection system shown in two different configurations. FIG. 5C depicts a front view of the disinfection system. And FIG. 5D depicts a side view of the disinfection system.
FIG. 6 schematically illustrates an example disinfection system including modular units, according to embodiments disclosed herein.
FIG. 7 schematically illustrates an example disinfection system including modular units and capable of forming a sealed chamber, according to embodiments disclosed herein.
FIGS. 8A and 8B illustrate two different configurations of energy sources disposed on modular units of a disinfection system, according to embodiments disclosed herein.
FIGS. 9A and 9B illustrate two different views of an example energy source of a disinfection system, according to embodiments disclosed herein. FIG. 9A depicts a view of the energy source covering a surface of a modular unit of the disinfection system, and FIG. 9B depicts a cross-sectional view of a portion of the modular unit, showing the energy source and other layers of the modular unit.
FIG. 10 schematically illustrates an example of a disinfection system, according to embodiments disclosed herein.
FIG. 11 schematically illustrates an example of a disinfection system, according to embodiments disclosed herein.
FIG. 12 is a perspective view of an example of a disinfection system including modular units, according to embodiments disclosed herein.
FIGS. 13A and 13B depict different views of a modular unit of the disinfection system shown in FIG. 11.
FIG. 14A depicts an enlarged view of a portion of a modular unit of the disinfection system shown in FIG. 11.
FIG. 14B depicts an enlarged view of a portion of a modular unit of the disinfection system shown in FIG. 11, with a portion of an outer housing of the modular unit removed to shown an interior support structure of the modular unit.
FIGS. 15A and 15B depict different views of the disinfection system shown in FIG. 11. FIG. 14A depicts a side view of the disinfection system, and FIG. 14B depicts a top view of the disinfection system.
FIG. 16 depicts a front view of the disinfection system shown in FIG. 11, including an example of medical equipment disposed within the disinfection system.
FIG. 17 depicts a perspective view of an example of a disinfection system including modular units, according to embodiments disclosed herein.
FIG. 18 is a perspective view of an example of a disinfection system including modular units, according to embodiments disclosed herein.
FIG. 19 is a front view of the disinfection system shown in FIG. 18.
FIG. 20 is a back view of the disinfection system shown in FIG. 18.
FIGS. 21A-21D depict different modular units of the disinfection system shown in FIG. 18.
FIG. 22 is a flow chart of a method of disinfecting using a disinfection system, according to embodiments not being part of the claimed subject -matter.
FIG. 23 is a flow chart of a method of assembly a disinfection system, according to embodiments not being part of the claimed subject -matter.
FIG. 24 is a flow chart of a method of disinfecting using a disinfection system, according to embodiments not being part of the claimed subject -matter.
FIG. 25 is a flow chart of a method of monitoring adherence to disinfection protocols and generating reports, according to embodiments not being part of the claimed subject matter.
FIG. 26 schematically illustrates a kit including modular units and other components of a disinfection system, according to embodiments not being part of the claimed subject matter.
FIG. 27 is a schematic illustration of an object indicator tag, according to embodiments described herein.
FIGS. 28A and 28B illustrate examples of object indicator tags, according to embodiments described herein.
FIG. 29 is a schematic illustration of a disinfection system connected via a network to one of more other disinfection systems and other compute devices, according to embodiments described herein.
FIG. 30 is a flow chart of a method of disinfecting using a disinfection system, according to embodiments not being part of the claimed subject -matter.

### DETAILED DESCRIPTION

Systems, apparatus, and methods described herein relate to disinfecting structures formed at least in part of modular units and/or components. Systems, apparatus, and methods disclosed herein can be designed to disinfect objects or areas using energy sources that emit light (e.g., UV light) at distances and intensities capable of disinfecting various surfaces and materials and/or disinfecting agents (e.g., hydrogen peroxide, peracetic acid, electrolyzed water, atmospheric pressure plasma, polymeric guanidine, ozone, or combinations thereof) in amounts capable of disinfecting various surfaces and materials. Systems, apparatus, and methods disclosed herein can be designed to disinfect, e.g., reduce the count of microorganisms (e.g., bacteria, viruses, etc.) from surfaces of objects, to various degrees, depending on requirements (e.g., set by a hospital or organization) and/or the nature or means of disinfection (e.g., the type of disinfection used, an amount of time for the disinfection, the object being disinfected, the distance of the object from the disinfecting source, etc.). For example, disinfection systems disclosed herein can be capable of disinfecting an object to a particular level (e.g., cleaning, sanitizing, low-level disinfecting, high-level disinfecting, sterilizing), depending on the classification of that object based on its risk of infection. Embodiments of disinfection systems can be designed to select a level of disinfection based on a type of object and/or area being disinfected and operate to disinfect accordingly.

Although embodiments of the present disclosure are described with specific reference to systems and methods for disinfecting medical equipment (e.g., gurneys, wheelchairs, intravenous (IV) poles, dialysis machines, etc.) or medical enclosures (e.g., hospital rooms, surgery suites, diagnostic laboratories, etc.), it should be appreciated that such systems and methods may be used to disinfect a variety of items used or contacted by the public (e.g., shopping carts, shopping baskets, strollers, railings, door knobs, etc.) and a variety of enclosures (e.g., kitchens, public or private bathrooms, cafeterias, airplanes, buses, etc.).

Systems, devices, and methods described herein can be integrated into an active workflow environment, e.g., of a hospital, a nursing home, a grocery store, a gym, or other facility. In some embodiments, systems, devices, and methods described herein can be designed to provide efficient and effective disinfection of objections (e.g., medical equipment and other high-contact objects), while ensuring safety of device operators and other users near the devices. In some embodiments, systems, devices, and methods described here can be designed to provide real-time analytics regarding a disinfection process, the operators, the objects being disinfected, and/or protocols.

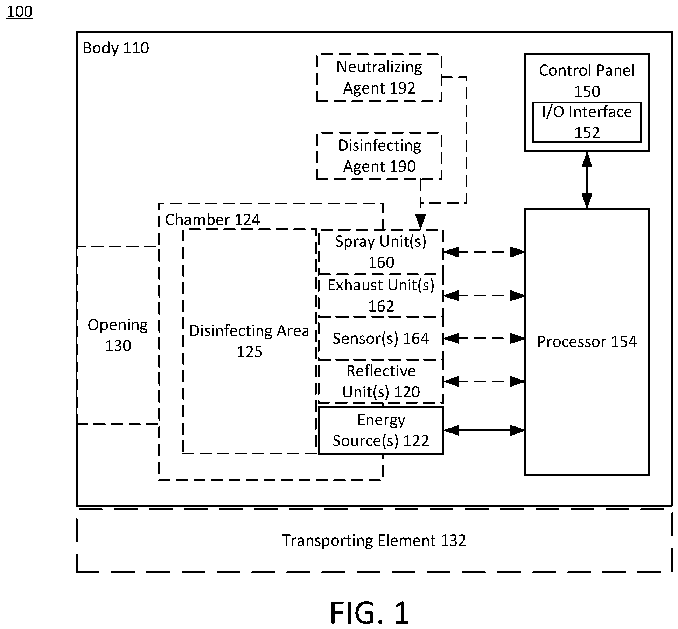

FIG. 1 is a high-level block diagram that schematically illustrates an example disinfection system 100, according some embodiments. Disinfection system 100 includes a body 110 and one or more energy source(s) 122. Disinfection system 100 can optionally include one or more reflective unit(s) 120, spray unit(s) 160, exhaust unit(s) 162, and/or sensor(s) 164.

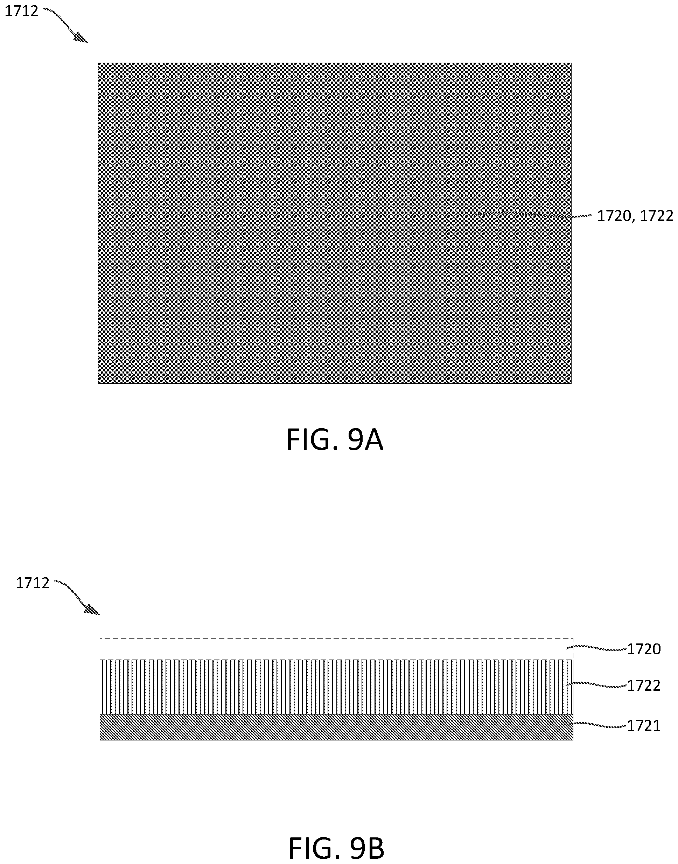

Each energy source 122 is configured to emit energy that can be directed at objects disposed within a disinfecting area 125. Each energy source 122 can be configured to emit light, such as, for example, UV light at a wavelength of approximately 320-400 nanometers (nm) (i.e., UV-A light), UV light at a wavelength of approximately 290-320 nm (i.e., UV-B light), UV light at a wavelength of approximately 100-280 nm (i.e., UV-C light), and/or high-intensity narrow-spectrum (HINS) light (e.g., light at a wavelength of 405 nm). In some embodiments, a first set of energy source(s) 122 can be configured to emit a first type of energy (e.g., UV-B light) and a second set of energy source(s) 122 can be configured to emit a second type of energy (e.g., UV-C light). Each energy source 122 can include one or more mercury vapor bulbs or tubes, xenon gas bulbs or tubes, excimer bulbs or tubes, light emitting diodes (LED), light emitting nanoparticles, lasers, or other energy sources configured to emit light. For example, energy source(s) 122 may include light bulbs that are configured to emit at least 30 watts of UV energy (e.g., 36 watts of UV energy). As another example, energy source(s) 122 may include light emitting nanoparticles deposited or grown on a flexible conductive layer, as further described below in reference to FIGS. 9A and 9B. As another example, energy source(s) 122 can be configured to emit HINS light.

One or more energy source(s) 122 can be disposed within (e.g., removably or permanently) or near a reflective unit 120, such that energy emitted from the energy source(s) 122 can be directed into a disinfecting area 125 and/or an object disposed within the disinfecting area 125. Each reflective unit 120 can be formed of one or more reflective surface(s) capable of reflecting energy emitted from the energy source(s) 122. For example, reflective unit(s) 120 can have a curved reflective surface (e.g., a hyperbolic reflective surface) that directs energy emitted from the energy source(s) 122 in multiple directions into the disinfecting area 125. Alternatively, reflective unit(s) 120 can have a back surface and a plurality of reflective surfaces disposed off normal with respect to the back surface that direct energy emitted from energy source(s) in multiple directions into disinfecting area 125. Reflective unit(s) 120 can include reflective materials, such as, for example, mirrors, powder-coated materials or metal sheets, or Pebbletone^{™} and Hammertone^{™} finishes.

Energy source(s) 122 can be configured to emit energy having an intensity at a predefined distance (e.g., 100 µW/c\m² at 1 meter) that is capable of disinfecting the surfaces of an object disposed within that predefined distance. In embodiments including reflective unit(s) 120, reflective unit(s) 120 can work in cooperation with energy source(s) 122 to ensure that a sufficient amount of energy for disinfecting an object is deposited on each surface of the object. Each surface of an object disposed within disinfecting area 125 can receive a collective amount of energy from various beams of energy (e.g., directly emitted by energy source(s) 122 and/or reflected via reflective unit(s) 120) that is sufficient to disinfect the surface, i.e., sufficiently reduce or eliminate pathogens disposed on the surface.

The disinfection system 100, including reflective unit(s) 120 and energy source(s) 122 as disposed around the disinfecting area 125, can be configured to increase (e.g., optimize) the effectiveness of a disinfection procedure, e.g., via intensity, proximity, and line of sight, by having objects within the disinfecting area 125 be fully enclosed in a highly reflective space. In some embodiments, disinfection system 100 (and other disinfection systems as described herein) can be configured to deliver a sufficient amount of disinfecting energy (e.g., UV-C light) in a predefined period of time to an object to disinfect the object (e.g., inactivate pathogens). The predefined period of time can be about 10 second, about 30 seconds, about 60 seconds, about 2 minutes, about 3 minutes, about 5 minutes, about 10 minutes, and including any values and/or sub-ranges there-between. In some embodiments, the disinfection system 100 can be configured to achieve a Log-5 reduction (e.g., 99.99% reduction) in pathogens in about 60 seconds or less. Examples of pathogens that the disinfection system 100 can be configured to reduce or eliminate include bacteria such as multi-drug resistant gram-positive bacteria (e.g., methicillin-resistant staphylococcus aureus (MRSA)), fungus such as multi-drug resistant fungus (e.g., *Candida auris*), viruses, microorganisms, etc. In some embodiments, disinfection system 100 can be configured to provide about 360 mJ/cm² of disinfecting energy within the disinfecting area 125 (e.g., at a center of the chamber 124). In some embodiments, the disinfection system 100 can be configured to provide greater than about 200 mJ/cm², greater than about 250 mJ/cm², greater than about 300 mJ/cm², greater than about 350 mJ/cm², greater than about 400 mJ/cm², or greater than about 500 mJ/cm², including all values and subranges in-between.

Disinfecting area 125 can be disposed adjacent to and/or within body 110 of disinfection system 100. For example, disinfecting area 125 can be located within a chamber 124 defined by body 110, or disinfecting area 125 can be an area that is adjacent to body 110. In some embodiments, the disinfecting area 125 can have dimensions of at least about 914.4mm (36") (W) x about 2032mm (80") (H) x about 1270mm (50") (D). In some embodiments, the disinfecting area 125 can have a width between about 508mm (20") to about 1016mm (40"), including all values and ranges in-between. In some embodiments, the disinfecting area 125 can have a width that is at least about 508mm (20"), at least about 762mm (30"), or at least about 1016mm (40"), including all values and ranges in-between. In some embodiments, the disinfecting area 125 can have a height between about 1778mm (70") to about 2286mm (90"). In some embodiments, the disinfecting area 125 can have a height that is at least about 1778mm (70"), at least about 2032mm (80"), or at least about 2286mm (90"), including all values and ranges in-between. In some embodiments, the disinfecting area 125 can have a length between about 1 016mm (40") to about 1524mm (60"), including all values and ranges in-between. In some embodiments, the disinfecting area 125 can have a length that is at least about 1016mm (40"), at least about 1270mm (50"), or at least about 1524mm (60"), including all values and ranges in-between.

Body 110 can optionally define a chamber 124 for receiving an object requiring disinfecting. In some embodiments, the body 110 or exterior of the disinfection system 100 can have dimensions of at most about 1244. 6mm (49") (W) x 2235. 2mm (88") (H) x 1524mm (60") (D). In some embodiments, the body 110 can have a width between about 762mm (30") to about 1524mm (60"), including all values and ranges in-between. In some embodiments, the body 110 can have a height between about 1778mm (70") to about 2540mm (100"), including all values and ranges in-between. In some embodiments, the body 110 can have a length between about 1270mm (50") to about 2032mm (80"), including all values and ranges in-between.

Chamber 124 can be sized to receive the object, and can include an opening 130 through which the object can be placed within chamber 124. The object can be, for example, medical equipment such as a gurney, a wheelchair, a pole to support bags of fluid for intravenous delivery (an IV pole), a medical cart, a mobile or portable computer station, a dialysis machine, an anesthesia machine, an electrocardiogram (ECG) machine, and/or other types of mobile medical items. Body 110 can include a wall, panel, and/or other structure capable of moving between an open configuration and a closed configuration to open and close the opening 130 of chamber 124. In some embodiments, chamber 124 can be designed to seal in energy and/or fluid, such that energy and/or fluid deposited within chamber 124 cannot exit chamber 124. Such sealing can prevent energy and/or fluid within the chamber 124 from affecting surrounding objects and/or persons, and allow use of certain types of disinfecting agents that may be harmful to surrounding objects and/or persons. Alternatively, in some embodiments, chamber 124 can be designed as an open chamber. For example, disinfection system 100 may include walls or surfaces that partially surround an open space (e.g., a disinfecting area 125). In such embodiments, any energy and/or disinfecting agents used with the disinfection system 100 may be ones that are not harmful to surrounding objects and/or persons. For example, a UV light source such as an excimer light source can be used to disinfect without objects in an open chamber without causing safety concerns.

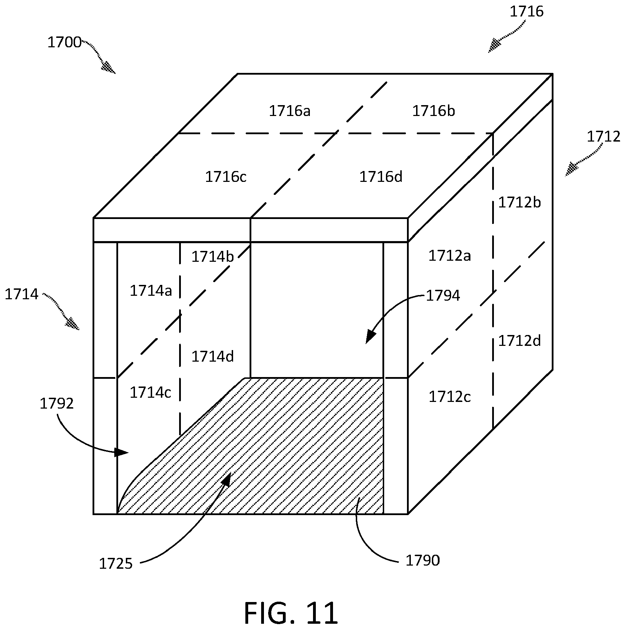

In some embodiments, disinfection system 100 can be designed with or for use with a conveyor unit or other type of transport unit configured to move an object being disinfected through the disinfecting area 125. For example, disinfection system 100 can include one or more side panels or walls that are directed at the disinfecting area 125, and a transport unit (e.g., conveyor belt) can be configured to move the object being disinfected through the disinfecting area 125. In an embodiment, disinfection system 100 can include two side walls and a top wall (each formed of one or more modular units, as further described below) that encircle a space for receiving objects, and a bottom wall with a conveyor belt positioned thereon for moving the objects through the space. As objects are moved through the space encircled by the walls, the objects can be disinfected by one or more energy source(s) 122, reflective unit(s) 120, and/or spray unit(s) 160. An example of a disinfection system with a conveyor unit is described in more detail with reference to FIG. 11.

Disinfection system 100 can optionally include a transporting element 132. Transporting element 132 can be any combination of suitable components configured for movement, such as, for example, a wheel, a caster, a rail, a skid, a sled, a track, etc. Transporting element 132 can be provided along a bottom or base of disinfection system 100 and can enable movement of disinfection system 100, e.g., within a medical facility. Suitable examples of disinfection systems including transporting elements are disclosed in U.S. Patent Application Publication No. 2017/0340760, titled "System for disinfecting larger scale spaces and equipment," filed May 23, 2017.

Disinfection system 100 can operate according to one or more disinfecting modes. Disinfection system 100 can be designed to vary the disinfecting mode based on user inputs and/or sensed information regarding an object, e.g., the location of the object relative to one or more energy source(s) 122, the dimensions of the object, the type of object, the materials of the object, whether the object has hard or soft surfaces, the required level of disinfection associated with the object, etc. For example, disinfection system 100 can be configured to vary an amount of time of disinfection, use a subset of available energy source(s) 122, use specific types of energy source(s) 122 (when multiple types are available), adjust the configuration and/or positioning of reflective unit(s) 120, etc.

In some embodiments, disinfection system 100 includes spray unit(s) 160 (e.g. fluid dispensers) for applying one or more agents (e.g., disinfecting agent 190, neutralizing agent 192) to objects within disinfecting area 125. Disinfection system 100 can use spray unit(s) 160 to apply the agents to further disinfect and/or treat an object being disinfected. Spray unit(s) 160 can be configured to dispense the agents in a liquid spray and/or a vapor / gas. In some embodiments, spray unit(s) 160 can be adjusted (e.g., via processor 154 and/or control panel 150) to change a direction and/or spray profile of a sprayed substance. For example, spray unit(s) 160 can include one or more nozzles with openings that can be adjusted to vary an amount of liquid and/or vapor that is sprayed, the profile of the produced spray, and/or a direction of the produced spray. In some embodiments, spray unit(s) 160 can apply an electrostatic charge to the sprayed agent to encourage droplets of the agent to spread out more evenly and adhere to the neutral or negative charged surfaces of objects. In some embodiments, spray unit(s) 160 can be connected to a source of pressurized gas that can be used to generate aerosolized streams of disinfecting agent 190 and/or neutralizing agent 192.

In some embodiments, disinfection system 100 can be configured to use one or more energy source(s) 122 to disinfect an object, as well as one or more spray unit(s) 160 to apply a disinfecting agent 190 and/or a neutralizing agent 192 to the object. By disinfecting with energy source(s) 122 and disinfecting agents 190, disinfection system 100 can target different types surfaces and/or different types of pathogens. For example, energy source(s) 122 capable of emitting UV-C light have been shown to be effective at killing pathogens on hard surfaces, while a disinfecting agent 190 such as hydrogen peroxide has been shown to be effect at disinfecting soft surfaces. Therefore, disinfection system 100 may use both energy source(s) 122 and disinfecting agents 190 to disinfect an object having hard and soft surfaces. Disinfection system 100 may run a first disinfection cycle using energy source(s) 122 that emit UV-C light (e.g., a UV-C cycle) and a second disinfection cycle using the disinfecting agents 190 (e.g., a vapor cycle), sequentially or simultaneously. In an embodiment, disinfection system 100 can be configured for photocatalytic disinfection. For example, disinfection system, via spray unit(s) 160, can apply a light-activated photosensitizer (e.g., titanium dioxide) to surfaces and use UV light and/or electromagnetic radiation emitted by one or more energy source(s) 122 to activate the photosensitizer and disinfect the surfaces.

Disinfecting agent 190 can include, for example, hydrogen peroxide, peracetic acid, electrolyzed water, atmospheric pressure plasma, polymeric guanidine, or ozone. Neutralizing agent 192 can be configured to reduce degradation of the object caused by the use of a disinfecting agent 190 and/or a particular type of energy source 122. For example, neutralizing agent 192 can be applied before, during, and/or after activing the energy source(s) 122 and/or applying the disinfecting agent 190 to treat the surfaces of the object being disinfected, such that the object degrades less over time. Neutralizing agent 192 can also be configured to reduce the risk of harmful contact between a human and a disinfecting agent 190. An example of a suitable neutralizing agent 192 can be water. Disinfecting agent 190 and/or neutralizing agent 192 can be delivered as a liquid spray and/or vapor.

Disinfection system 100 can optionally include exhaust unit(s) 162 configured to vent away air from disinfecting area 125 (e.g., to vent air out of chamber 124) and/or supply clean air to disinfecting area 125 (e.g., to supply clean air into chamber 124). Exhaust unit(s) 162 can be used in conjunction with spray unit(s) 160 to vent away air and/or vapors carrying disinfecting agent 190 and/or neutralizing agent 192. When disinfection system 100 is used with spray unit(s) 160 and/or exhaust unit(s) 162, body 110 can define a sealed chamber (e.g., chamber 124) such that air containing disinfecting agents, neutralizing agents, and/or other substances can be sealed within the chamber and removed via exhaust unit(s) 162, without exposing a user outside of the disinfecting area 125 to such air.

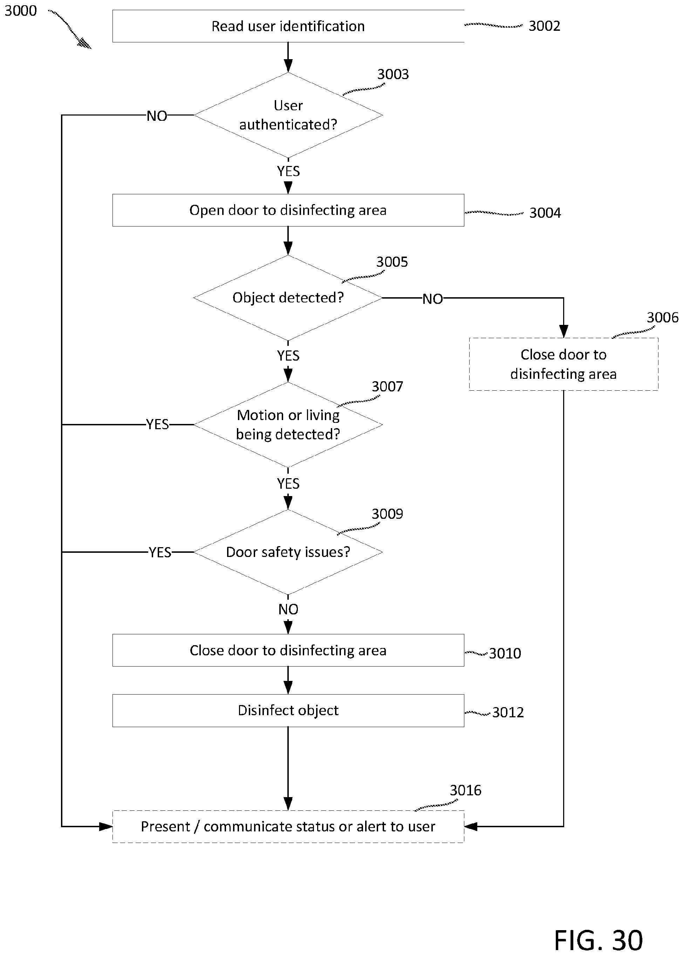

In some embodiments, disinfection system 100 includes one or more sensor(s) 164 for collecting information regarding components of disinfection system 100, objects within and/or near disinfection system 100, and/or other information that may affect the operation of disinfection system 100. Sensor(s) 164 can be coupled and/or integrated into a panel or wall of disinfection system 100 or another component of disinfection system 100 (e.g., an energy source 122, a reflective unit 120, a spray unit 160, or an exhaust unit 162). Sensor(s) 100 can include, for example, motion sensors, image capture devices (e.g., cameras), light sensors, temperature sensors, pressure sensors, sound detectors, ozone sensors, etc. For example, sensor(s) 164 can include at least one motion sensor capable of detecting movement within and/or near disinfecting area 125 to determine whether a user may be harmed by energy, disinfecting agents, and/or other components of disinfection system 100. Alternatively or additionally, sensor(s) 164 can be configured to monitor for other safety issues, e.g., door obstruction, door closure pinching, etc. Further details of such safety monitoring sensors are described with reference to FIG. 30. In some embodiments, sensor(s) 164 can be configured to detect and/or determine information about objects within disinfecting area 125. For example, sensor(s) 164 can include image capture devices that can capture images of objects within disinfecting area 125 and determine the object type, dimensions of the objects, distance and/or positioning of the objects relative to energy source(s) 122 and/or spray unit(s) 160, or other information regarding the objects. Alternatively or additionally, sensor(s) 164 can include weight sensors, light sensors, etc. located on a base or floor associated with disinfection system, which can measure the weight, position, size, and/or orientation of objects within disinfecting area 125. Disinfection system 100 can use this information to determine how to disinfect the objects. In some embodiments, sensor(s) 164 can be coupled to and/or integrated into an energy source 122, reflective unit 120, and/or a spray unit 160, and be configured to monitor the operation of such components. For example, sensor(s) 164 can monitor temperatures and/or moisture levels associated with such components, which can be used to confirm and/or modify disinfecting procedures.

In some embodiments, sensor(s) 164 can include a radio frequency identification (RFID) sensor, Quick Response (QR) reader, or other type of suitable sensor for identifying a user of the disinfection system 100 and/or objects placed within the disinfecting area 125. Such sensor(s) 164 can be used to control access to and/or use of the disinfection system 100, and/or to log information associated with use of the disinfection system 100. For example, a RFID sensor can be configured to read a badge or other identifying card and/or device of a user to permit access by the user to the disinfection system 100. A RFID sensor or a QR reader can be configured to read a tag (e.g., a RFID tag or QR code) located on an object placed in the disinfecting area 125 to identify the type of object. In some embodiments, the sensor(s) 164 can include a multi-frequency RFID sensor and/or Bluetooth-compatible badge reader, which can be configured to read multiple types of access cards and badges. In some embodiments, a badge reader can include encryption functionality, such that existing badges of an institution (e.g., a hospital) can be used with the disinfection system 100. An onboard processor (e.g., processor 154, described below) or another device in communication with the sensor(s) 164, e.g., via a wired or wireless connection, can log the disinfections performed by the disinfection system 100. In some embodiments, sensor(s) 164 can also detect when maintenance of the disinfection system 100 may be required, e.g., when a sensor 164 detects that a particular energy source 122 may no longer be functional.

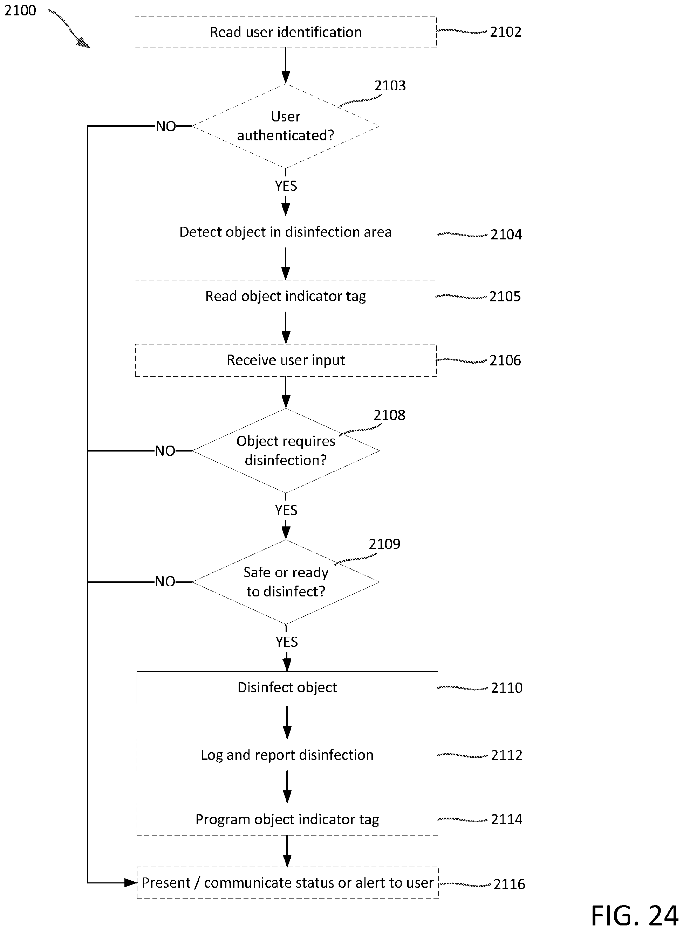

Disinfection system 100 can include a control panel 150 with an input/output (I/O) interface 152. Control panel 150, via I/O interface 152, can be configured to receive and process user inputs and/or monitor the operations and functions of disinfection system 100. The control panel 150 can be electrically coupled to a processor 154, which can be used to control one or more components of disinfection system 100. Processor 154 can be any suitable processing device configured to execute functions associated with disinfection system 100. For example, processor 154 can be configured to activate one or more energy source(s) 122. In embodiments where disinfection system 100 includes reflective unit(s) 120, processor 154 can be electrically coupled to one or more reflective unit(s) 120 and be configured to move (e.g., rotate, translate, etc.) the reflective unit(s) 120 to control the direction that energy is reflected, e.g., to target a surface of an object and/or specific area within disinfecting area 125. In embodiments where disinfection system 100 includes sensor(s) 164, processor 154 can be electrically coupled to one or more sensor(s) 164 and be configured to receive information from the sensor(s) 164 (e.g., information regarding objects within disinfecting area 125, one or more components of disinfection system 100, and/or an environment around disinfection system 100). Processor 154 can be configured to select specific disinfection modes (e.g., disinfection type or cycle), deactivate the disinfection system 100 (e.g., in cases where a user may be harmed by disinfection procedures), change a disinfection step, and/or provide an alert or status update (e.g., an audio and/or visual alert, or an electronic alert), based on the information received from the sensor(s) 164. Processor 154 can be, for example, one or more of a general purpose processor, a Field Programmable Gate Array (FPGA), an Application Specific Integrated Circuit (ASIC), a Digital Signal Processor (DSP), and/or the like. Disinfection system 100 can also include an onboard power source (e.g., a battery) and/or be coupled to a power source (e.g., be plugged into a wall socket). I/O interface 152 can include a user interface with one or more components that are configured to receive inputs and/or present outputs to users and/or user devices. For example, the user interface can include a display device (e.g., a display, a touchscreen (e.g., glove-compatible touchscreen), etc.), an audio device (e.g., a microphone, a speaker), a keyboard, a scanner or reader (e.g., a radio-frequency identification (RFID) reader, a near-field communication (NFC) reader, etc.), etc. In some embodiments, the user interface (e.g., touchscreen or other display) can be configured to provide instructions to a user, e.g., step-by-step instructions that instruct a user on how to operate the disinfection system 100, e.g., how to open the door, place an object inside the disinfection area, close the door, activate the disinfection, remove the object after the disinfection, close the door after removing the object, etc. Such steps can involve, for example, scanning a badge, pressing a button, providing an input to a control panel (e.g., a touchscreen). In some embodiments, the user interface (e.g., touchscreen or other display) can be configured to provide error notifications, operational metrics, safety alerts, etc. While not specifically depicted in FIG. 1, disinfection system 100 can include a memory or storage device that can store information regarding the disinfection system and one or more disinfection cycles that are run by the disinfection system 100 (e.g., time and date of running disinfection cycles, user or operator that initiated the disinfection cycle, type of disinfection, object being disinfected, any errors or alerts associated with the disinfection, etc.). Further details of a disinfection cycle and information that can be logged by the disinfection device 100 are described with reference to FIGS. 24, 25, and 30.

In some embodiments, disinfection system 100 can be connected to a network (e.g., a local area network (LAN), a wide area network (WAN), a virtual network, a telecommunications network) implemented as a wired network and/or wireless network and be configured to communicate with other devices coupled to the network, e.g., another disinfection system 100, a server, or other compute devices. In some embodiments, disinfection system 100 can include a multi-network modem that can be configured to connect via LAN, cellular, Wi-Fi, etc. to one or more networks. Disinfection system 100 can be configured to receive and send information via the network, including, for example, information regarding the operation of and/or disinfections performed by disinfection system 100. In some embodiments, disinfection system 100, via the network, can be connected to a remote control panel or system through which a user can remotely control disinfections system 100. In some embodiments, disinfection system 100 can be connected to a cloud network that hosts one or more other applications that can interface with disinfection system 100 to provide other service(s). For example, disinfection system 100 can be connected to a remote server that tracks the object(s) that have been disinfected by the disinfection system 100, and can provide this information to administrator and/or for reporting purposes. In some embodiments, disinfection system 100 can also report information regarding disinfected object(s) to cloud-based applications and/or devices that facilitate real-time updates to local staff within a medical facility (e.g., updates at a computer and/or electronic indicator tags on disinfected object(s)).

Disinfection system 100 can be a unitary structure, or disinfection system 100 can be implemented as multiple structures located in the vicinity of one another. In some embodiments, disinfection system 100 can be formed of modular units that can be assembled together to form disinfection system 100, as further described below.

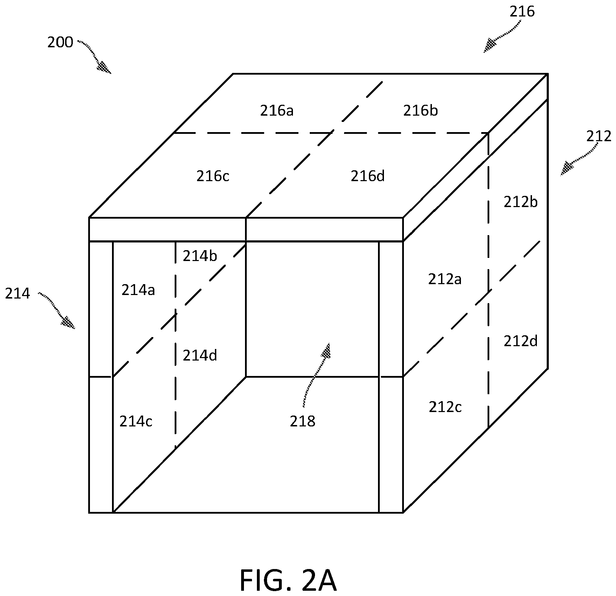

FIG. 2A depicts an example disinfection system 200, according to embodiments disclosed herein. Disinfection system 200 can be formed of a plurality of modular units. Disinfection system 200 includes side walls 212, 214, a top wall 216, and a back wall 218. In some embodiments, disinfection system 200 can also include additional walls, e.g., a floor or bottom wall and/or a front door or wall (not depicted). Each wall 212, 214, 216, 218 can be formed of one or more modular units. For example, wall 212 can optionally be formed of four modular units 212a, 212b, 212c, 212d; wall 214 can optionally be formed of four modular units 214a, 214b, 214c, 214d; and wall 216 can be formed of four modular units 216a, 216b, 216c, 216d. Alternatively, each of walls 212, 214, 216 can be formed of a single modular unit. Modular units 212a, 212b, 212c, 212d can be similar to one another and include the same components (e.g., energy source(s), reflective unit(s), spray unit(s), etc.) and/or also be similar to other modular units of other walls, e.g., modular units 214a, 214b, 214c, 214d and/or modular units 216a, 216b, 216c, 216d. Each modular unit can be designed to be interchangeable with one or more other modular units, e.g., modular unit 212a can be interchangeable with any one of modular units 212b, 212c, 212d and/or other modular units, such as one or more of modular units 214a, 214b, 214c, 214d, 216a, 216b, 216c, 216d.

Each modular unit can be manufactured and/or assembled at a manufacturing facility and transported to a location for onsite assembly into disinfection system 200. Transportation costs can be reduced by transporting the modular units separately to an onsite location. Individual modular units can be dimensioned to fit through standard sized doorways and openings within a building, such as, for example, a medical facility. Each modular unit can also weigh and/or be dimensioned such that a human can directly lift and/or move the units, or use standard moving tools to lift and/or move the units, such as, for example, a dolly, a lift, a moving cart, etc.

Each modular unit can be coupled to the modular units adjacent to it via suitable fastening elements (e.g., mechanical fasteners, magnets, adhesives, etc.). In some embodiments, modular units can include built-in connectors for quick coupling and assembly, e.g., snap-on connectors, magnetic connectors, etc.

While disinfection system 200 is depicted as a box-shaped structure, in other embodiments, disinfection system can have a different shape, e.g., a spherical shape, a pyramidal shape, a cylindrical shape, etc.

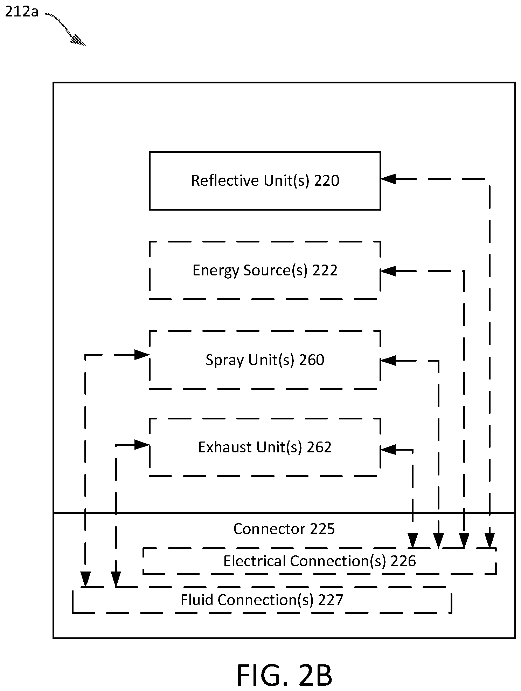

FIG. 2B provides a schematic view of modular unit 212a, which can be similar to and/or the same as other modular units depicted in FIG. 2A (e.g., modular units 212b, 212c, 212d, 214a, 214b, 214c, 214d, 216a, 216b, 216c, 216d). Modular unit 212a can include at least one reflective unit 220 and can optionally include one or more energy source(s) 22, spray unit(s) 260, and/or exhaust unit(s) 262. Reflective unit 220 can be similar to reflective unit 120, as described above. For example, reflective unit 220 can have one or more surfaces configured to reflect energy emitted by energy sources disposed on modular unit 212a and/or other modular units (e.g., modular units 212b, 212c, 212d, 214a, 214b, 214c, 214d, 216a, 216b, 216c, 216d). When modular unit 212a is assembled in disinfection system 200, reflective unit 220 can be disposed on an inner surface of wall 212, such that reflective unit 220 can be configured to direct energy emitted by energy sources into a chamber defined by walls 212, 214, 216. In some embodiments, reflective unit 220 can be adjusted, e.g., manually and/or automatically, to change the direction that it directs energy into the chamber defined by walls 212, 214, 216. Reflective unit 220 can have a concave shape (e.g., a hyperbolic shape) that can spread and reflect energy into the chamber, or have multiple reflective surfaces that are angled with respect to one another to spread and reflect light into the chamber. In some embodiments, reflective unit 220 can be implemented as a reflective coating that can cover a portion or all of an inner facing surface of modular unit 212a. In other embodiments, reflective unit 220 can be implemented as a one or more reflective surface mounted on beams or other support structures attached to an inside surface of modular unit 212a.

Energy source(s) 222 can be similar to energy source(s) 122. For example, each energy source 222 can be configured to emit energy, such as, for example, UV light or HINS light. When modular unit 212a is assembled in disinfection system 200, energy source(s) 222 can be disposed on an inner surface of wall 212, such that energy emitted by energy source(s) 222 can be directed into the chamber defined by walls 212, 214, 216. In some embodiments, energy source(s) 222 can emit light that is predominantly UV-C light (e.g., at least 75% of which is UV-C light).

While reflective unit(s) 220 and energy source(s) 222 are described with reference to a modular unit 212a, one or more reflective unit(s) 220 and/or energy source(s) 222 can be disposed on other modular units, e.g., one or more of modular units 212b, 212c, 212d, 214a, 214b, 214c, 214d, 216a, 216b, 216c, 216d, or on other surfaces of disinfection system 200 (e.g., back wall 218 and/or a bottom wall or floor). Collectively, reflective unit(s) 220 and/or energy source(s) 222 disposed on the modular units, walls, and/or other portions of disinfection system 200 can ensure that an adequate amount of energy reaches each surface of an object that is located within the chamber, such that the object can be disinfected using the emitted energy. More specifically, reflective unit(s) 220 and/or energy source(s) 222 can be disposed in any suitable location, orientation, configuration, size, and/or number such that an object within the chamber defined by walls 212, 214, 216 is exposed to energy at sufficient intensities (e.g., at least 100 µW/cm² at 1 meter) for a sufficient amount of time to enable disinfection.

Spray unit(s) 160 can be similar to spray unit(s) 160. For example, spray unit(s) 260 can be configured to deliver one or more agents (e.g., disinfecting agents, neutralizing agents) into the chamber. When modular unit 212a is assembled in disinfection system 200, spray unit(s) 260 can be disposed on an inner surface of wall 212, such that spray unit(s) 260 can direct one or more agents at an object located within the chamber. Spray unit(s) 260 can be configured to deliver the agents as a liquid spray and/or vapor. In some embodiments, spray unit(s) 260 can be configured to electrically charge droplets of the agent such that the droplets are predisposed to evenly distribute and/or adhere to the surfaces of an object within the chamber. Modular unit 212a can include fluid connection(s) 227 that are coupled to spray unit(s) 260 and can provide fluid communication between spray unit(s) 260 and a source of an agent (e.g., a fluid reservoir). Fluid connection(s) 227 can include ports and/or channels integrated into, coupled to, and/or coupleable to the modular unit 212a.

Exhaust unit(s) 262 can be similar to exhaust unit(s) 162. For example, exhaust unit(s) 262 can be configured to circulate air into and/or out of the chamber. For example, exhaust unit(s) 262 can be configured to vent air out of chamber, e.g., with a fan, by applying a vacuum or suction, or other suitable means. Additionally or alternatively, exhaust unit(s) 262 can circulate clean air into the chamber, e.g., via a fan, air pump, or other suitable means. Exhaust unit(s) 262 can be coupled to fluid connection(s) 227, which provide an inlet and/or outlet path for air from the chamber. In some embodiments, exhaust unit(s) 262, via fluid connection(s) 227, can vent air through a filtration and/or air purification system, which can clean and/or disinfect the air for recirculation through other fluid channels and/or exhaust unit(s) 262 back into the chamber.

Modular unit 212a can optionally include electrical connection(s) 226. Electrical connection(s) 226 can be coupled to one or more of reflective unit(s) 220, energy source(s) 222, spray unit(s) 260, and/or exhaust unit(s) 262, to connect those components to a power source and/or control unit (e.g., an onboard or off-board control unit, including, for example, a processor and/or control panel). The control unit (not depicted) can be used to control the operation of one or more of reflective unit(s) 220, energy source(s) 222, spray unit(s) 260, and/or exhaust unit(s) 262. For example, the control unit can be used to selectively activate, move, and/or adjust one or more of one or more of reflective unit(s) 220, energy source(s) 222, spray unit(s) 260, and/or exhaust unit(s) 262. In some embodiments, the control unit can be coupled to one or more sensors for detecting information about the objects within chamber, the surrounding environment, and/or a user of the disinfection system 200.

Modular unit 212a can include a connector 225, which can be used to couple modular unit 212a to other modular unit(s) (e.g., modular unit 212b or 212c). In some embodiments, connector 225 can include electrical connection(s) 226 and/or fluid connection(s) 227, which can be coupled to one or more of reflective unit(s) 220, energy source(s) 222, spray units) 260, and/or exhaust unit(s) 262. Connector 225 can be disposed on a side of modular unit 212a, such that connector 225 can be configured to couple to a connector on an adjacent modular unit (e.g., modular unit 212b or 212c) when the two modular units are attached to one another. In some embodiments, connector 225 can be designed as a snap-on connector that can engage with a corresponding connector located on an adjacent modular unit. Connector 225, via a network of connections (e.g., including additional connectors, electrical connection(s), and/or fluid connection(s)) through modular units, can be coupled to an air ventilation system, an air filtration system, a source of disinfecting agent and/or neutralizing agent, a control unit, power source, etc.

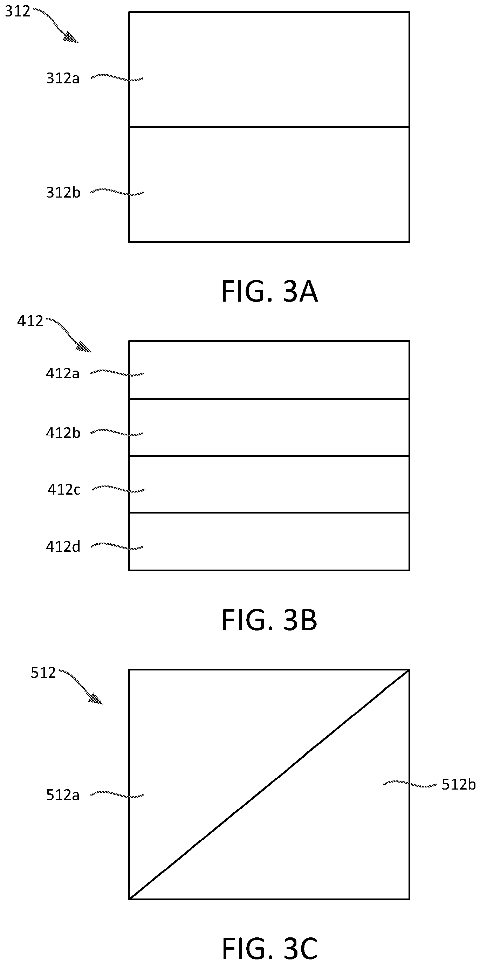

While disinfection system 200 is depicted as having walls that can be formed of a single modular unit or, optionally, formed of four modular units placed in a two-by-two arrangement (e.g., wall 212 being formed of four modular units 212a, 212b, 212c, 212d), other disinfection systems can include walls with different arrangements and/or configurations (e.g., shapes, sizes, etc.) of modular units. For example, FIG. 3A depicts a wall 312 formed of two modular units 312a, 312b that are rectangular-shaped positioning in a two-by-one arrangement (i.e., with modular unit 312a positioned above modular unit 312b). FIG. 3B depicts a wall 412 formed of four modular units 412a, 412b, 412c, 412d that are rectangular-shaped and positioned in a four-by-one arrangement. FIG. 3C depicts a wall 512 formed of two modular units 512a, 512b, each having a triangular shape and coming together to form a rectangular shaped wall. Different arrangements and/or configurations of modular units can be used, e.g., to accommodate differently shaped components (e.g., energy sources, reflective units, spray units, exhaust units) and/or due to space limitations (e.g., during shipping and transport to an onsite location). In some embodiments, modular units can be designed with longer coupling surfaces (e.g., for coupling to adjacent modular units), such that more connections between the two modular units can be accommodated and/or more stability can be provided via the coupling.

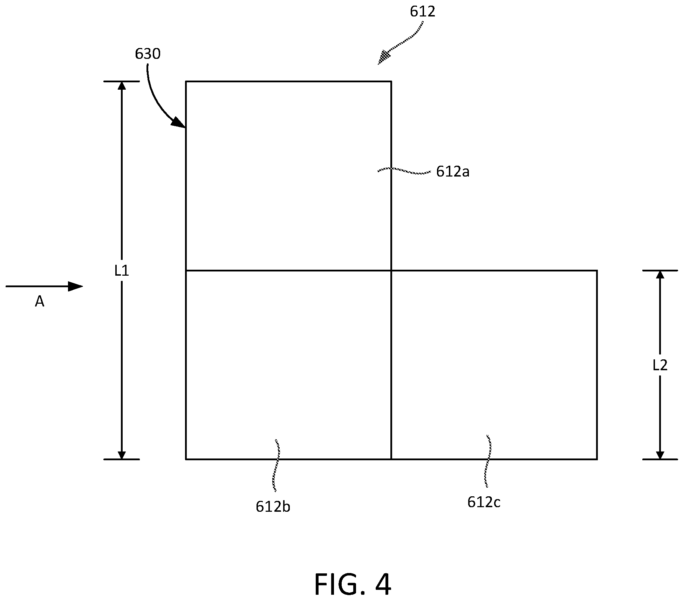

FIG. 4 depicts another arrangement of modular units forming a wall 612 of a disinfection system. As depicted, wall 612 is formed of three modular units 612a, 612b, 612c. Modular units 612a, 612b, 612c can similar to and/or the same as one another. Modular units 612a, 612b, 612c can be arranged in a L-shaped configuration, such that wall 612 can have ends with varying lengths. Specifically, wall 612 can have a first end with a length L1 and a second end with a length L2, where length L1 is equal to the combined length of two modular units 612a, 612b and length L2 is equal to the length of a single modular unit 612c. When wall 612 is assembled in a disinfection system, wall 612 can define a chamber that has regions with varying height such that it can receive a similarly shaped object. For example, the chamber can be configured to receive a gurney with an attached IV pole such that the height of the IV pole can be accommodated by the taller region defined by modular units 612a, 612b. Other arrangements and/or configurations of modular units can be used to construct disinfection systems capable of receiving objects having other sizes and/or shapes.

Disinfection systems having a modular design can have improved customizability, adaptability, and/or serviceability. For example, individual modular units can be designed to be interchangeable with one another, and can be assembled together in a number of different ways to form differently shaped and/or sized disinfection systems. Depending on a user's disinfection needs and/or space limitations, the user can select from several different types of modular units, and can assemble those modular units in various ways to define differently sized and shaped disinfection areas and/or chambers. When a particular component of a modular unit requires maintenance and/or repair, that modular unit can be replaced without requiring the entire disinfection system to be serviced and/or replaced, thereby reducing repair costs and/or downtime. Additionally, when improvements to modular units are available (e.g., a new design of a particular modular unit becomes available), existing disinfection systems can be outfitted with the new modular units by replacing old units with the new ones without requiring a full redesign and/or replacement of the system.

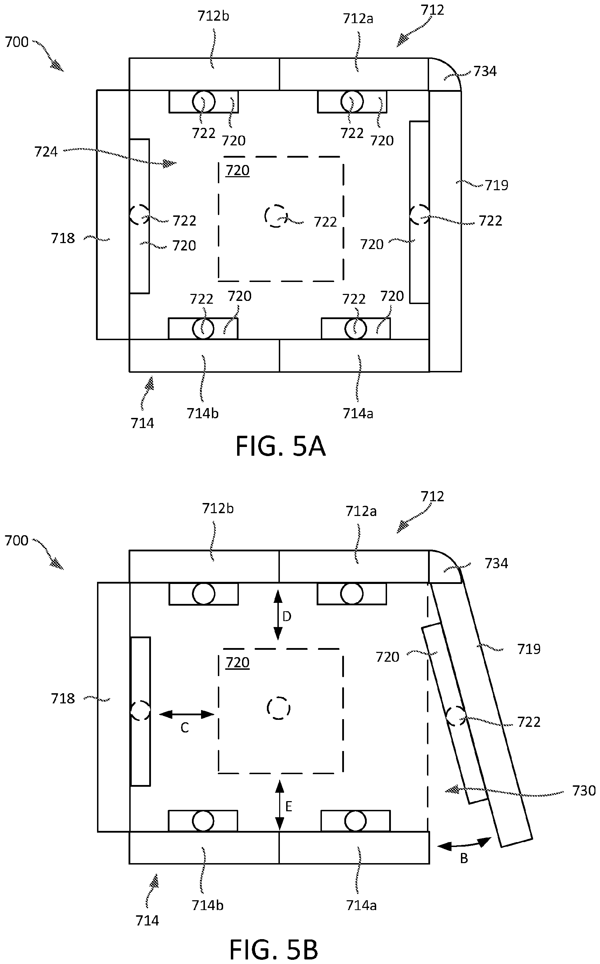

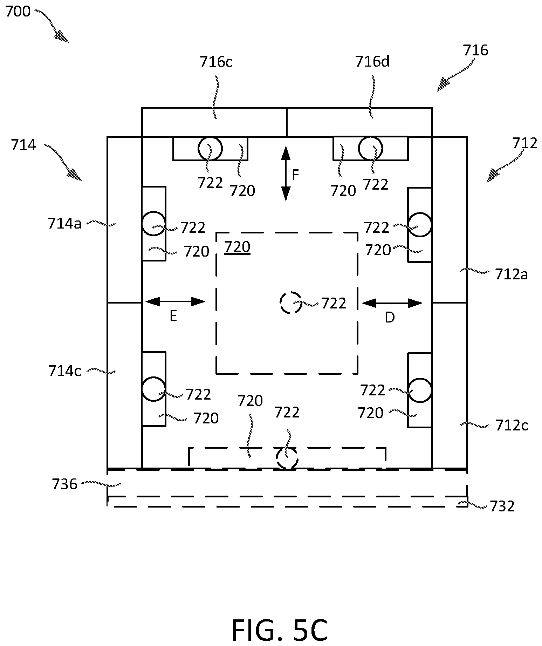

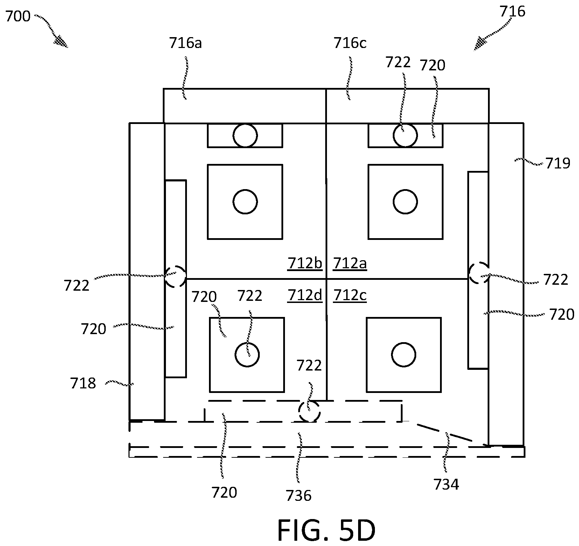

FIGS. 5A, 5B, 5C, and 5D schematically illustrate an example disinfection system 700, according to some embodiments. Disinfection system 700 includes a plurality of walls, i.e., side walls 712, 714, a top wall 716, a back wall 718, a front wall 719, and optionally a base or bottom wall 736. FIGS. 5A and 5B depict a top view of disinfection system 700, with the top wall 716 removed to show interior features of the system. FIG. 5C depicts a front view of the disinfection system 700, with the front wall 719 removed to show interior features of the system. And FIG. 5D depicts a side view of the disinfection system 700, with the side wall 714 removed to show interior features of the system.

Walls 712, 714, 716, 718, 719, 736 can define a chamber 724 sized to receive objects for disinfection. While walls 712, 714, 716, 718, 719, 736 are depicted as extending substantially perpendicular from one another to define a rectangular-shaped chamber 724, it can be appreciated that disinfection systems described herein can have different configurations and/or shapes. For example, any one of walls 712, 714, 716 can extend at an off-normal axis from back wall 718, and can have a non-rectangular shape (e.g., a trapezoidal shape, a triangular shape, etc.). Walls 712, 714, 716 can define an opening 730 through which an object can be received within chamber 724.

Front wall 719 can be movable between a closed configuration (as depicted in FIG. 5A) and an open configuration (as depicted in FIG. 5B). Front wall 719 can move between the closed configuration and the open configuration, as shown via arrow B. Front wall 719 can be coupled to side wall 712 via a joint 734, and can pivot about joint 734 to move between the closed configuration and the open configuration. While front wall 719 is shown as a single panel, in other embodiments, front wall 719 can be formed of multiple panels and/or sections, and each panel can be coupled to the same or a different side wall. An example of such an arrangement is described below with reference to FIGS. 11-15. In some embodiments, front wall 719 can be coupled to side wall 712 (and/or other side walls) using a mechanical connector other than a joint, e.g., using an elastic material, a slider bar, etc. In some embodiments, disinfection system 700 can include a control unit that can electrically operate front wall 719, e.g., to move it between an open configuration and a closed configuration. Front wall 719 can be formed of a rigid and/or flexible material (e.g., metal, cloth, fabric, plastic, etc.). In some embodiments, front wall 719 can be designed to be retractable, such that it can be retracted to expose opening 730.

When front wall 719 is in the closed configuration, front wall 719 can seal opening 730 of chamber 724 such that energy (e.g., UV light) within chamber 724 does not exit chamber 724. In some embodiments, walls 712, 714, 716, 718, 719, 736 can form an air-sealed chamber such that air (e.g., air including disinfecting agent and/or neutralizing agent and/or contaminated air) cannot exit chamber 724. By sealing in energy and/or air, front wall 719 can reduce the risk of a user outside of chamber 724 from being harmed by energy and/or air present within chamber 724. When front wall 719 is in the open configuration, front wall 719 can allow access to opening 730 such that an object can be placed within chamber 724 via opening 730.

One or more of walls 712, 714, 716, 718, 719, 736 can be formed of modular units. For example, wall 712 can be formed of four modular units 712a, 712b, 712c, 712d; wall 714 can be formed of four modular units 714a, 714b, 714c, 714d; and wall 716 can be formed of four modular units 716a, 716b, 716c, 716d. In some embodiments, one or of more walls 718, 719, 736 can also be formed of modular units, including some walls being formed of a single modular unit.

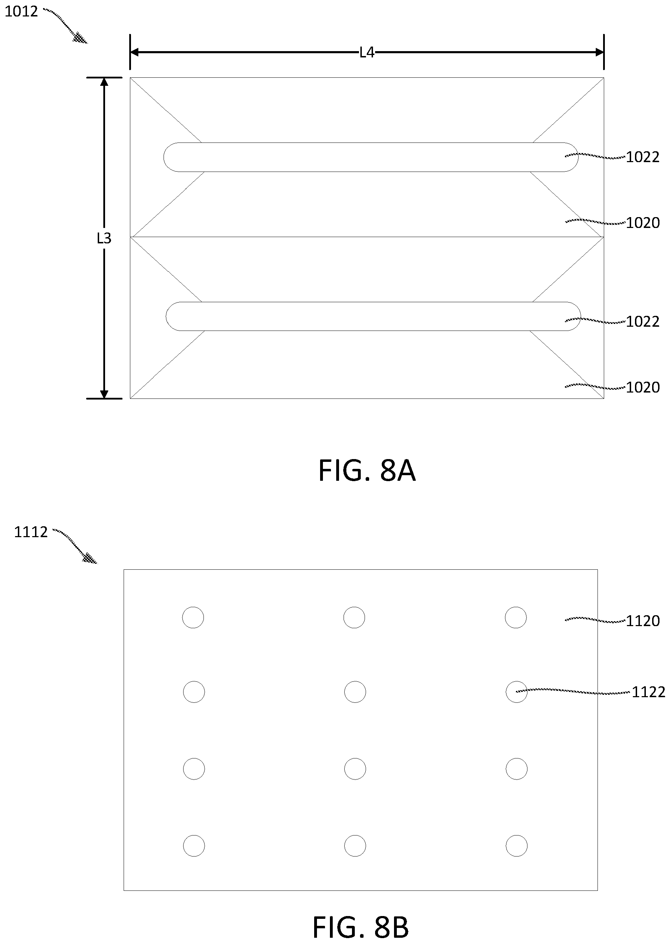

Modular units 712a, 712b, 712c, 712d, 714a, 714b, 714c, 714d, 716a, 716b, 716c, 716d can be designed to be interchangeable with one another. Therefore, each modular unit can have similar components as other modular units. An inner surface of each modular unit can include at least one reflective unit 720 and at least one energy source 722. Additional reflective unit(s) 720 and/or energy source(s) 722 can be positioned on the inside surfaces of walls 718, 719, 736, as shown in FIGS. 5A-5D. For example, an inner surface of wall 718 can include at least one reflective unit 720 and, optionally, one or more energy source(s) 722. An inner surface of wall 719 can include at least one reflective unit 720 and, optionally, one or more energy source(s) 722. And an inner surface of wall 736 can include at least one reflective unit 720 and, optionally, one or more energy source(s) 722. In an embodiment, each of modular units 712a, 712b, 712c, 712d, 714a, 714b, 714c, 714d, 716a, 716b, 716c, 716d can include at least one least one reflective unit 720 and at least one energy source 722, and each of walls 718, 719, 736 can include at least one reflective unit 720. While reflective unit(s) 720 and/or energy source(s) 722 are depicted on a central portion of the modular units, it can be appreciated that reflective unit(s) 720 and/or energy source(s) 722 can be located on any portion of an inner surface of a modular unit and/or cover an entire inner surface of a modular unit. And while reflective unit(s) 720 are depicted as surrounding energy source(s) 722, it can be appreciated that reflective unit(s) 720 and/or energy source(s) 722 can be positioned in any suitable arrangement, including arrangements where reflective unit(s) 720 and energy source(s) 722 each cover an entire inner surface of a modular unit. Further examples of these and other arrangements of reflective unit(s) 720 and/or energy source(s) 722 are described below with reference to FIGS. 8A, 8B, 9A, and 9B.

Reflective unit 720 can be similar to other reflective units described herein (e.g., reflective units 120 and/or 220). For example, reflective unit 720 can include one or more reflective surfaces that can reflect energy (e.g., disinfecting light emitted by an energy source 722). In some embodiments, reflective unit 720 can be implemented as a reflective coating and/or flat reflective surface. Energy source 722 can be similar to other energy sources described herein (e.g., energy source 122 and/or 222). For example, energy source 722 can be configured to emit a light capable of disinfecting a surface of an object, such as, for example, UV light and/or HINS light. Each energy source 722 can be disposed near and/or within a reflective unit 720, such that energy emitted by the energy source 722 can be reflected and directed into chamber 724 via the reflective unit 720. Other energy sources 722 and/or reflective units 720 located on other modular units can also emit and reflect energy into chamber 724, such that a collective amount of energy sufficient for disinfection is directed into chamber 724 and/or at an object disposed within chamber 724.

In some embodiments, one or more modular units 712a, 712b, 712c, 712d, 714a, 714b, 714c, 714d, 716a, 716b, 716c, 716d, or walls 712, 714, 716, 718, 719, 736 can be movable relative to other components of disinfection system 700. For example, wall 712 and/or an individual modular unit 712a, 712b, 712c, 712d can be movable along an axis D; wall 714 and/or an individual modular unit 714a, 714b, 714c, 714d can be movable along an axis E; wall 716 and/or an individual modular unit 716a, 716b, 716c, 716d can be movable along an axis F; and wall 718 can be movable along an axis C. Additionally or alternatively, one or more reflective unit(s) 720 and/or energy source(s) 722 can be movable relative to a wall or modular unit. Movement of walls, modular units, reflective unit(s) 720, and/or energy source(s) 722 can be controlled by a control unit (e.g., a processor and/or control panel, such as processor 154 and/or control panel 150), and/or be moved manually by a user (e.g., via a mechanical mechanism, such as a lever, and/or by directly pushing or pulling on an individual component). Walls, modular units, reflective unit(s) 720, and/or energy source(s) 722 may be moved to position reflective unit(s) 720 and/or energy source(s) 722 closer to an object within chamber 724 to increase the efficiency of the disinfection process. For example, if a small object is placed within chamber 724, one or more walls, modular units, reflective unit(s) 720, and/or energy source(s) 722 may be moved to reduce the distance between the object and reflective unit(s) 720 and/or energy source(s) 722 and/or a size of the overall chamber 724, such that a greater intensity of energy is received at the surfaces of the object. Increasing the intensity of the energy received by the object can reduce disinfection time and/or improve disinfection efficacy.

While axes C, D, E, F are shown as being perpendicular to walls 718, 712, 714, 716, respectively, one of ordinary skill in the art would appreciate that walls, modular units, reflective unit(s) 720, and/or energy source(s) 722 can move in other directions, such as, for example, rotate about an axis and/or translated in angled directions.

Optionally, in some embodiments, disinfection system 700 can include a transporting element 732, such as, for example, wheels, casters, sleds, tracks, etc. Transporting element 732 may be disposed along a bottom surface of wall 736. Transporting element 732 can be similar to other transporting elements described herein (e.g., transporting element 132). For example, transporting element 732 can enable disinfection system 700 to maneuver through spaces, including rooms within a medical facility. In some embodiments, transporting element 732 can be retracted into openings formed in wall 736.

In some embodiments, wall 736 can include a ramped surface 734 to facilitate placement of an object into chamber 724 and/or removal of an object form chamber 724. In some embodiments, wall 736 can also include elements for guiding an object to a specific location within chamber 724 and/or restricting movement of the object within the chamber, such as, for example, stoppers, tracks, treads, depressions, etc. These elements can optionally be adjusted (e.g., via a processor and/or control panel, such as processor 154 and/or control panel 150) to accommodate different types of objects and/or change a placement of an object within chamber 724.

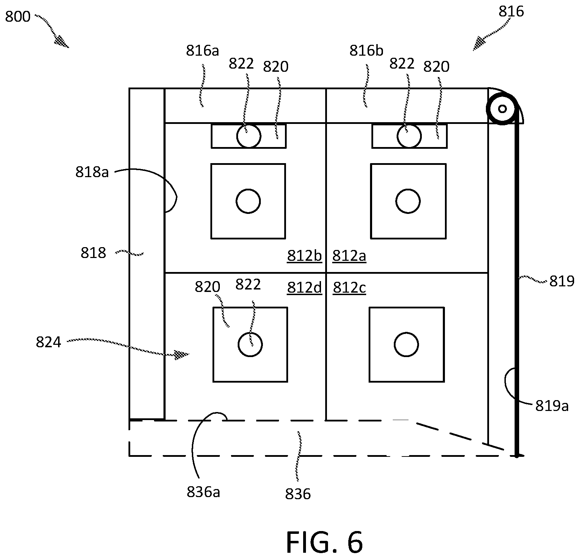

FIG. 6 schematically depicts a side view of an example disinfection system 800, according to other embodiments disclosed herein. Disinfection system 800 can include similar components as other disinfection systems described herein (e.g., disinfection systems 100, 200, and/or 700). Disinfection system 800 can include side walls (including a side wall 812), a top wall 816, a back wall 818, and optionally a bottom wall 836. In FIG. 6, a second side wall similar to wall 812 is not depicted so that an interior of disinfection system 800 can be viewed.

At least one wall of disinfection system 800 can be formed of one or more modular units. For example, side wall 812 can be formed of modular units 812a, 812b, 812c, 812d; and top wall 816 can be formed of at least two modular units 816a, 816b. Optionally, back wall 818 and/or bottom wall 836 can be formed of one or more modular units. Modular units 812a, 812b, 812c, 812d, 816a, 816b can each include at least one reflective unit 820 and at least one energy source 822. In some embodiments, back wall 818 can include a reflective inner surface 818a, and/or bottom wall 836 can include a reflective inner surface 836a. Reflective inner surfaces 818a, 836a can be implemented as a reflective coating and/or material that can reflect energy emitted by energy source(s) 822 into chamber 824. In some embodiments, inner surfaces 818a, 836a can also include an energy source configured to emit energy, such as, for example, light emitting nanoparticles. Further details regarding reflective surfaces including light emitting nanoparticles are described with reference to FIGS. 9A and 9B.

Disinfection system 800 can include a flexible curtain or drape 819 as a front wall. Curtain 819 can be configured to move between an open configuration and a closed configuration. In the closed configuration, as depicted in FIG. 6, curtain 819 can cover an opening to chamber 824. In the open configuration, curtain 819 can be retracted (e.g., rolled up) via a wheel or pulley, and/or pulled aside using another mechanical and/or electrical mechanism, to expose the opening to chamber 824 such that an object can be placed within chamber 824. Curtain 819 can include an inner surface 819a that can be a reflective surface. In some embodiments, inner surface 819a can also include at least one energy source, e.g., light emitting nanoparticles.

In some embodiments, one or more walls, modular units, reflective unit(s) 820, and/or energy source(s) 822 can be movable such that an angle or direction of reflective unit(s) 820 and/or energy source(s) 822 can be adjusted and/or a distance of reflective unit(s) 820 and/or energy source(s) 822 to an object be reduced.

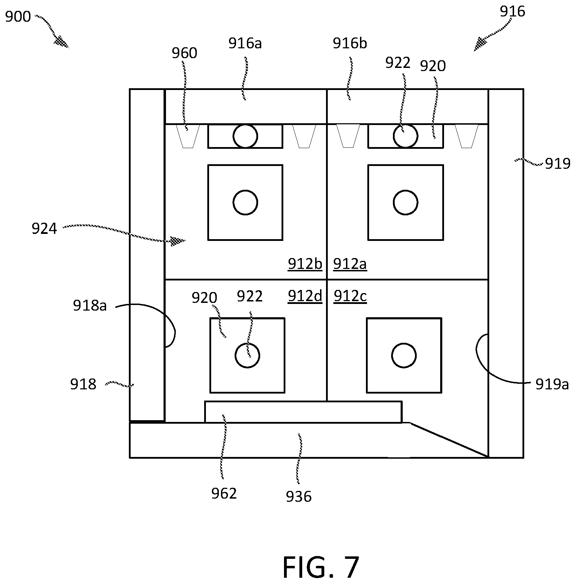

FIG. 7 schematically depicts a side view of an example disinfection system 900, according to other embodiments disclosed herein. Disinfection system 900 can be similar to disinfection system 700 (and include components that are similar to other disinfection systems described herein), but also include at least one spray unit 960. Disinfection system 900 can include side walls (including a side wall 912), a top wall 916, a back wall 918, and a bottom wall 936. In FIG. 7, a second side wall similar to wall 912 is not depicted so that an interior of disinfection system 900 can be viewed.

At least one wall of disinfection system 900 can be formed of modular units. For example, side wall 912 of disinfection system can be formed of modular units 912a, 912b, 912c, 912d, and top wall 916 of disinfection system can be formed of at least two modular units 916a, 916b. Modular units 912a, 912b, 912c, 912d, 916a, 916b can each include at least one reflective unit 920 and at least one energy source 922. In some embodiments, one or more walls can include a reflective inner surface, e.g., back wall 918 can include a reflective inner surface 918a and/or front wall 919 can include a reflective inner surface 919a.

At least one wall and/or modular unit of disinfection chamber 900 can include a spray unit 960. For example, modular units 916a, 916b can each include spray unit(s) 960. Spray unit(s) 960 can be similar to spray unit(s) 160, as described above. For example, spray unit(s) 960 can be configured to deliver substances, including, for example, a disinfecting agent, a neutralizing agent, and/or a photosensitizer. Spray unit(s) 960 can be configured to deliver such substances as a liquid spray and/or a vapor. Spray unit(s) 960 can be located on any portion of an inner surface of a modular unit and/or wall. In some embodiments, spray unit(s) 960 can be adjusted (e.g., via a processor and/or control panel, such as processor 154 and/or control panel 150) to change a direction and/or spray profile of a sprayed substance. In some embodiments, spray unit(s) 960 can apply an electrostatic charge to the sprayed substance to encourage droplets of the substance to spread out more evenly and adhere to the neutral or negative charged surfaces of an object within chamber 924.

Walls of disinfection chamber 900 can be designed to form a fluidically sealed chamber 924 that can prevent energy and/or other substances (e.g., a disinfecting agent, a neutralizing agent, and/or a photosensitizer) from exiting chamber 924. In some embodiments, spray unit(s) 960 can be configured to deliver a single type of disinfecting agent. In other embodiments, some spray units 960 can be configured to deliver a first type of disinfecting agent while other spray units 960 can be configured to deliver a second type of disinfecting agent, e.g., in the case where different disinfecting agents may be required to kill different types of pathogens. In other embodiments, some spray units 960 can be configured to deliver a disinfecting agent while other spray units 960 can be configured to deliver a neutralizing agent, e.g., in the case where a neutralizing agent may be used to reduce the degradation effects caused by the disinfection agent. In other embodiments, some spray units 960 can be configured to deliver a disinfecting agent while other spray units 960 can be configured to deliver a photosensitizer, e.g., in the case where a photosensitizer and a disinfecting agent may be used, along with an energy source (e.g., a UV light source), to disinfect an object.

Disinfection system 900 includes at least one exhaust unit 962. Exhaust unit 962 can be similar to exhaust unit 162, described above. For example, exhaust unit 962 can be configured to circulate air into and/or out of chamber 924, including air containing a disinfecting agent and/or a neutralizing agent. Exhaust unit 962 can be disposed on an inner surface of bottom wall or base 936. Alternatively or additionally, one or more exhaust units can be disposed on other walls and/or modular units of disinfection system 900.

In some embodiments, one or more walls, modular units, reflective unit(s) 920, energy source(s) 922, and/or spray unit(s) 960 can be movable such that an angle or direction of reflective unit(s) 920, energy source(s) 922, and/or spray unit(s) 960 can be adjusted and/or a distance of reflective unit(s) 920, energy source(s) 922, and/or spray unit(s) 960 to an object be reduced.

FIGS. 8A, 8B, 9A, and 9B depict different configurations of modular units having one or more energy sources and/or reflective units. FIG. 8A depicts an example modular unit 1012 having two reflective units 1020 and two energy sources 1022. Modular unit 1012 can be similar to any of the modular units described herein (e.g., modular units forming a part of disinfection systems 200, 700, 800, and/or 900). Each energy source 1022 can be housed within a reflective unit 1020. Energy sources 1022 can be implemented as light bulbs or tubes, such as, for example, a mercury vapor bulb or tube, a xenon gas bulb or tube, etc. In an embodiment, each energy source 1022 can be configured to emit UV light having an intensity of at least 100 µW/cm² at 1 meter. Each reflective unit 1020 can have a concave shape such that energy emitted from each of the respective energy sources 1022 can be directed and/or focused by the reflective unit 1020 toward a disinfecting area (e.g., into a chamber) and/or an object disposed in the disinfecting area. In an embodiment, each reflective unit 1020 can have a plurality of reflective surfaces that are disposed off normal with respect to a back section of the reflective unit 1020, such that energy emitted by each of the respective energy sources 1022 can be directed in multiple directions toward a disinfecting area and/or an object disposed in the disinfecting area.

Modular unit 1012 can be shaped as a panel with a width L3 and a length L4. The width L3 and the length L4 of modular unit 1012 can be appropriately sized for treating medical equipment and/or other objects within a medical facility (e.g., a hospital). Additionally, modular unit 1012 can be dimensioned to fit through standard sized doorways and openings in medical facilities, such that the units can easily be moved between rooms. In an embodiment, modular unit 1012 can have a width L3 of approximately 25 inches and a length L4 of approximately 50 inches. Multiple modular units, such as modular unit 1012, can be stacked relative to one another to form a disinfection system having dimensions for receiving various medical equipment and/or other objects. Examples of different arrangements of modular units are described with reference to FIGS. 11-16.

FIG. 8B depicts an example modular unit 1112 having a plurality of energy sources 1122. Energy sources 1122 can be arranged on a reflective surface 1120 acting as a reflective unit. Reflective surface 1120 can be a flat and/or curved surface that directs and/or focuses light toward a disinfecting area and/or an object located in disinfecting area. Reflective surface 1120 can be formed of a reflective material and/or include a reflective coating. Energy sources 1122 can be LEDs that are configured to emit UV and/or HINS light. Similar to modular unit 1012, modular unit 1112 can be sized to disinfect medical equipment or other objects located within a medical facility.

FIGS. 9A and 9B depict an example modular unit 1712 that includes a light source implemented as light emitting nanoparticles 1722. The light emitting nanoparticles 1722 can be deposited or grown on a flexible conductive layer. In an embodiment, molecular beam epitaxy (MBE) can be used to deposit nanowire heterostructures (e.g., GaN nanowires, AlGaN nanowires, InGaN nanowires) onto a conductive layer, such as, for example, a metal foil or film (e.g., a titanium foil, a tantalum foil, etc.). The nanowires may grow in arrays along the conductive layer surface. When energized (e.g., excited), the nanowires may emit energy at wavelengths between 350-400 nm. FIG. 9B provides a cross-sectional view of a portion of modular unit 1712, showing a layer of the light emitting nanoparticles 1722 deposited on a conductive layer 1721 (e.g., a metal foil or film). Suitable examples of light emitting nanoparticles are described by Sarwar et al. in "Semiconductor Nanowire Light-Emitting Diodes Grown on Metal: A Direction Toward Large-Scale Fabrication of Nanowire Devices," published August 25, 2015, available at https://doi.org/10.1002/smll.201501909, and "Nanowire LEDs Grown Directly on Flexible Metal Foil," available at https://aip.scitation.org/doi/am-pdf/10.1063/1.4945419.

Optionally, a reflective layer 1720, such as a thin coat or film, can be deposited on top of the light emitting nanoparticles 1722. Reflective layer 1720 can be a partially reflective and partially transparent element. Specifically, reflective layer 1720 can be configured to allow energy emitted by light emitting nanoparticles 1722 located below reflective layer 1720 to pass through but reflect energy directed at the reflective layer 1720 in the opposite direction. Reflective layer 1720 and light emitting nanoparticles 1722 can be positioned around a disinfecting area and/or a chamber such that light emitted by nanoparticles 1722 and/or reflected by reflective layer 1720 can be directed at the disinfecting area and/or an object located in the disinfecting area. For example, when modular unit 1712 is assembled in a disinfection system having a chamber, reflective layer 1720 and light emitting nanoparticles 1722 can be located on an inside surface of the modular unit 1712 that faces the chamber such that it can direct energy into the chamber and/or at an object located in the chamber.

Similar to modular unit 1012, modular unit 1712 can be sized to disinfect medical equipment or other objects located within a medical facility.

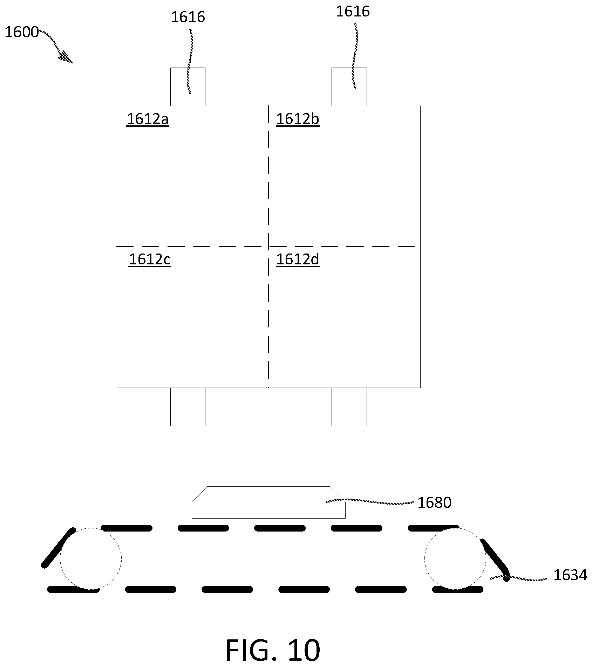

FIG. 10 depicts an example disinfection system 1600. Disinfection system 1600 can include similar components as other disinfection systems disclosed herein (e.g., disinfection systems 100, 200, 700, 800, and/or 900). Disinfection system 1600 can be formed of a plurality of modular units (e.g., modular units 1612a, 1612b, 1612c, 1612d), but the modular units do not form an enclosure that defines a chamber. Instead, disinfection system 1600 can be a wall-mounted or free-standing system that can focus and/or direct light at objects located in a disinfecting area (e.g., an object 1680). Modular units 1612a, 1612b, 1612c, 1612d can be supported and orientated by one or more support elements 1616 (e.g., a beam, a rod, a platform, etc.). Modular units 1612a, 1612b, 1612c, 1612d can be similar to other modular units described herein (e.g., modular unit 212a), and can include components such as a connector, an energy source, a reflective unit, a spray unit, an exhaust unit, and/or a sensor.

Disinfecting area can be, for example, a section of a room that is closed off using curtains or other barriers. Objects, such as object 1680, can be transported into the disinfecting area via a transport device 1634, such as, for example, a moving track. Alternatively, objects can be placed in disinfecting area by a user and/or a mechanical and/or electrical device (e.g., a robotic device).

Disinfection system 1600 can have a processor and/or control panel (not depicted) configured to control the operation of disinfection system 1600. Optionally, disinfection system 1600 can also have other components, e.g., a source of disinfecting agent, neutralizing agent, etc., and/or a transporting element, such as described with reference to disinfection system 100 depicted in FIG. 1.

FIG. 11 depicts an example disinfection system 1700. Disinfection system 1700 can include similar components as other disinfection systems disclosed herein (e.g., disinfection systems 100, 200, 700, 800, 900, and/or 1600), and additionally include or be designed for use with a transport unit 1790. Disinfection system 1700 can be formed of one or more modular units. For example, disinfection system 1700 can include side walls 1712, 1714 and a top wall 1716 that can be optionally formed of one or more modular units. For example, wall 1712 can optionally be formed of four modular units 1712a, 1712b, 1712c, 1712d; wall 1714 can optionally be formed of four modular units 1714a, 1714b, 1714c, 1714d; and wall 1716 can be formed of four modular units 1716a, 1716b, 1716c, 1716d. Alternatively, each of walls 1712, 1714, 1716 can be formed of a single modular unit, two modular units, or any other number of modular units. One or more modular units 1712a, 1712b, 1712c, 1712d, 1714a, 1714b, 1714c, 1714d, 1716a, 1716b, 1716c, 1716d can be similar to other modular units, e.g., include the same components (e.g., energy source(s), reflective unit(s), spray unit(s), etc.).

Each modular unit can be manufactured and/or assembled at a manufacturing facility and transported to a location for onsite assembly into disinfection system 1700. Each modular unit can be coupled to the modular units adjacent to it via suitable fastening elements (e.g., mechanical fasteners, magnets, adhesives, etc.). In some embodiments, modular units can include built-in connectors for quick coupling and assembly, e.g., snap-on connectors, magnetic connectors, etc.

As depicted in FIG. 11, disinfection system 1700 does not include a front wall or a back wall. Rather, side walls 1712, 1714 and top wall 1716 define an open disinfecting area 1725. Disinfection system 1700 can include a transport device 1790 that transport objects through the disinfecting area 1725. For example, objects can be placed on the transport device 1790 on a first side of the disinfection system 1700 and be transported through a first opening 1792 on the first side into the disinfecting area 1725, and out through a second opening 1794 on a second side of the disinfection system 1700. While the object is being transported through the disinfecting area 1725, the object can be disinfected by one or more energy source(s), reflective unit(s), and/or spray unit(s) located on the modular units of the disinfection system 1700.

The transport device 1790 can be any suitable device for moving an object through an area. For example, the transport device 1790 can be a conveyor belt that extends along a bottom side of the disinfection unit 1790. Alternatively or additionally, the transport device 1790 can include robotic components (e.g., robotic arms, manipulators, etc.) configured to couple to the objects (e.g., grab, magnetically couple, etc.) and move them through the disinfecting area 1725 (e.g., by lifting, pulling, etc.). In some embodiments, the transport device 1790 can be designed to re-position an object (e.g., using tracks, flippers, manipulators, etc.) prior to or during movement of the object through the disinfecting area 1725.

Since the disinfecting area 1725 is not entirely sealed from the surrounding environment, disinfection system 1700 can be designed for use with energy sources and/or disinfecting agents that are not harmful to surrounding objects and/or persons. For example, disinfection system 1700 can include light sources that are excimer lamps that emit far UV-C light, e.g., light having a wavelength of approximately 222 nm.

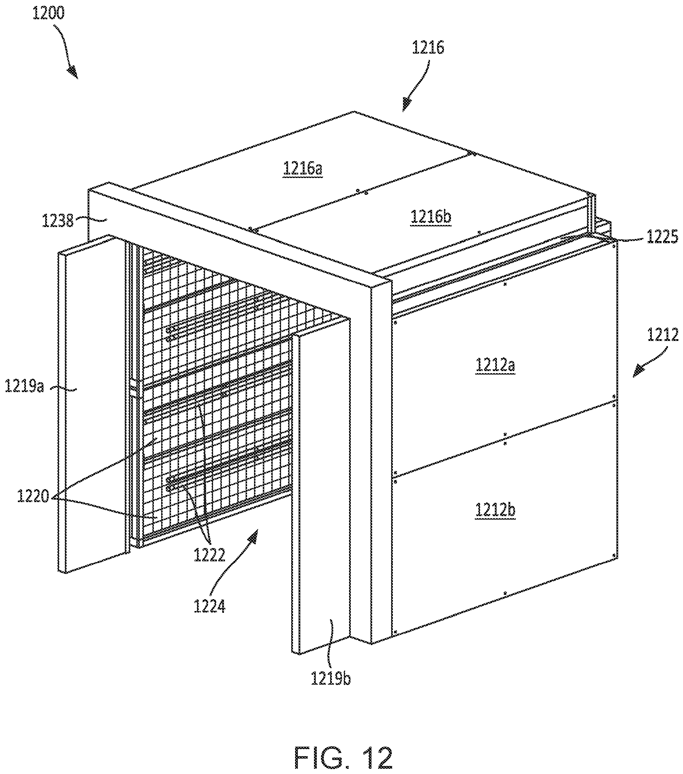

FIGS. 12-16 illustrate different views of an example disinfection system 1200. Disinfection system 1200 can be similar to other disinfection systems described herein (e.g., disinfection systems 100, 200, 700, 800, and/or 900), and can include similar components as those systems. Disinfection system 1200 includes a plurality of walls, including side walls 1212, 1214, a top wall 1216, and a back wall 1218. Each wall 1212, 1214, 1216, 1218 can be formed from two modular units. Specifically wall 1212 is formed of modular units 1212a, 1212b; wall 1214 is formed of modular units 1214a, 1214b; wall 1216 is formed of modular units 1216a, 1216b; and wall 1218 is formed of modular units 1218a, 1218b.

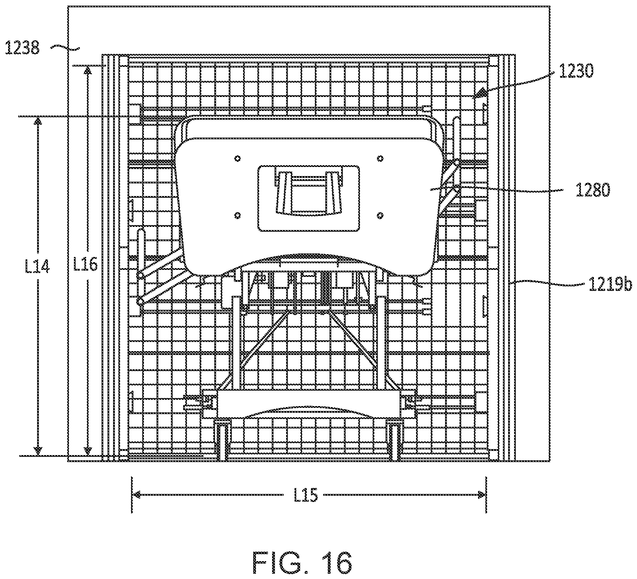

Disinfection system 1200 also includes two panel sections 1219a, 1219b that can open and close. Panel sections 1219a, 1219b can be two bi-folding doors. When panel sections 1219a, 1219b are in an open configuration, as best shown in FIGS. 12 and 16, panel sections 1219a, 1219b provide an opening 1230 into a chamber 1224 defined by walls 1212, 1214, 1216, 1218. When panel sections 1219a, 1219b are in a closed configuration, panel sections 1219a, 1219b seal off opening 1230 such that energy and/or fluids (e.g., air, liquid, vapor) within chamber 1224 cannot exit chamber 1224. Each panel section 1219a, 1219b can fold into its open configuration (as shown in FIGS. 12 and 16) and unfold into its closed configuration. Panel sections 1219a, 1219b can be mounted to a support frame 1238, which can be coupled to modular units 1212a, 1212b, 1214a, 1214b, 1216a, 1216b. When panel sections 1219a, 1219b are in the open configuration, opening 1230 has a length L15 and a height L16, as shown in FIG. 16. While two panel sections 1219a, 1219b are shown, one of ordinary skill in the art would appreciate that other suitable components for closing and opening a chamber opening can be used, such as, for example, a single door that can pivot open and close, a single bi-folding door that can fold and unfold to open and close, a pair of doors that can pivot open and close, a retractable curtain, etc.

In some embodiments, panels sections 1219a, 1219b can be configured to automatically open and/or close, e.g., in response to a user identifying themselves (e.g., by scanning a badge, mobile device, or other device near a NFC or RFID reader). For example, a user desiring to disinfect an object within the disinfection system 1200 can scan a badge and, in response to the disinfection system 1200 verifying an identify of the user and determining that the user is authorized to use the disinfection system 1200, the disinfection system 1200 (e.g., via a processor such as, for example, processor 154) can automatically cause the panels sections 1219a, 1219b to open to permit the user to place an object within the chamber 1224. In some embodiments, after the object is placed within the chamber 1224 and/or after a predefined period of time elapsing since the panels sections 1219a, 1219b opening, the disinfection system 1200 (e.g., via the processor) can cause the panels sections 1219a, 1219b to automatically close. In some embodiments, the disinfection system 1200 can include additional sensors (e.g., sensor(s) 164) within the chamber 1224 that can detect that an object is within the chamber, and then initiate a disinfection process, as further described herein. Accordingly, in use, the disinfection system 1200 can be enabled for "no touch" operation, e.g., be configured to operate without requiring a user to touch any portion of the disinfection system 1200.

Each modular unit 1212a, 1212b, 1214a, 1214b, 1216a, 1216b, 1218a, 1218b can be connected to adjacent modular units via one or more connectors 1225. Connectors 1225 can be attachable to and/or integrated into one or more modular units. For example, connectors 1225 can include snap-on components that can mate with one another to connect two modular units together. Alternatively or additionally, connectors 1225 can include fasteners, adhesives, magnets, etc. that can adhere two adjacent modular units to one another. In some embodiments, connectors 1225 can include electrical connections and/or fluid connections, which can connect to electrical connections and/or fluid connections in adjacent modular units such that a network of electrical connections and/or fluid connections can be formed to connect one or more components of modular units 1212a, 1212b, 1214a, 1214b, 1216a, 1216b, 1218a, 1218b to power source(s), fluid source(s), a control panel, a processor, and/or other centralized elements.

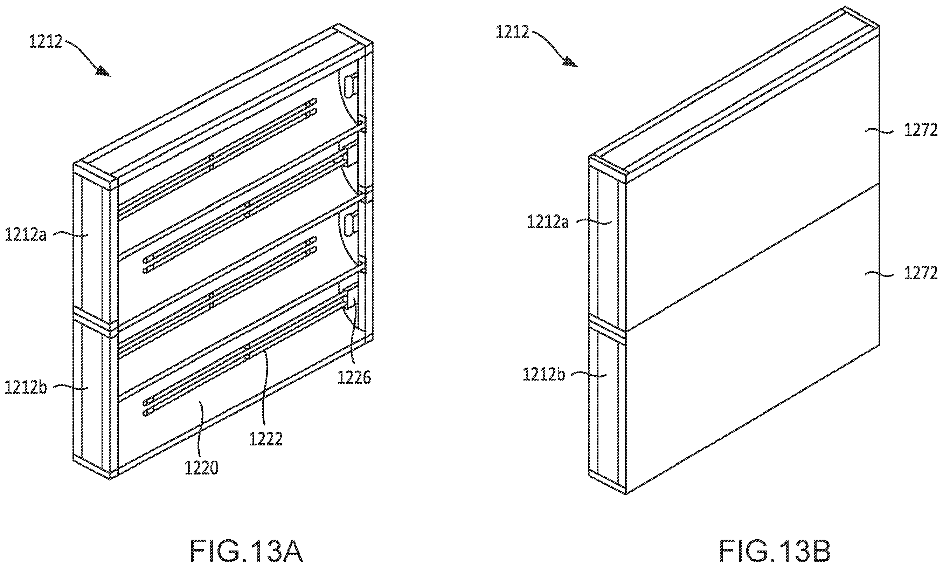

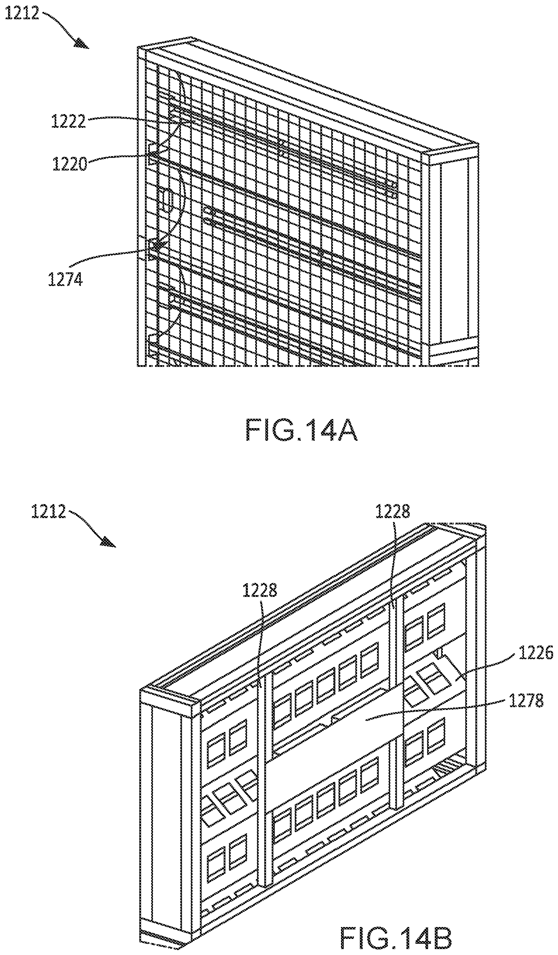

FIGS. 13A, 13B, 14A, and 14B provide detailed views of modular units 1212a, 1212b of wall 1212. While not depicted in detail, other modular units of disinfection system 1200 (e.g., modular units 1214a, 1214b, 1216a, 1216b, 1218a, 1218b) can be identical to and/or similar to modular units 121a, 1212b. Each modular unit 1212a, 1212b includes two reflective units 1220 and two energy sources 1222. Reflective units 1220 and/or energy sources 1222 can be similar to other reflective units and energy sources described herein. For example, each reflective unit 1220 can have a reflective surface that can reflect energy emitted by energy sources 1222 into chamber 1224. As depicted in FIGS. 13A, 13B, 14A, and 14B, each reflective unit 1220 can have a hyperbolically shaped reflective surface that can be configured to distribute energy emitted by energy sources 1222 and reflect it into chamber 1224. Each energy source 1222 can be disposed within a reflective unit 1220. Energy sources 1222 can include at least one light tube configured to emit disinfecting light (e.g., UV light). Energy sources 1222 can be connected to electrical connectors 1226 disposed within or adjacent to each reflective unit 1220.

In some embodiments, additional reflective units 1220 and/or energy sources 1222 can be disposed on an inner surface of a front wall (e.g., panels 1219a, 1219b) and a bottom side of chamber 1214. For example, reflective material (e.g., a coating and/or flat sheet) can be placed on panels 1219a, 1219b and/or a floor of chamber 1224 and function as reflective units 1220. Reflective units 1220 and energy source 1222, when assembled around chamber 1224 and operating together, can be configured to collectively deliver a sufficient amount of light at a sufficient intensity onto the surfaces of an object within chamber 1224, such that the object can be adequately disinfected.

Each modular unit includes at least one removable cover or panel (e.g., panels 1272, 1274) to allow reflective units 1220 and/or energy sources 1222 to be inspected, repaired, or replaced. For example, FIG. 13A provides a view of modular units 1212a, 1212b with panels 1274 removed, and FIG. 14B provides a view of a portion of modular unit 1212a with panel 1272 removed. Removal of any one of panels 1272, 1274 can expose an internal structure of the modular unit and provide access to reflective units 1220 and/or energy sources 1222. The internal support structure can include one or more vertical support elements 1228 and/or horizontal support elements 1278. A back portion 1226 of reflective units can be mounted to one or more support elements 1228, 1278. In some embodiments, additional components (e.g., energy sources, spray units, etc.) can also be mounted directly to a support element. Panels 1272, 1274 can be configured to protect reflective units 1220 and/or energy sources 1222, as well as other internal components of the modular units.

In some embodiments, each modular unit can be easily detached from its adjacent modular units and removed from the disinfection system 1200 for inspection, repair, and/or replacement. In some embodiments, removal of a single modular unit does not compromise the overall structure of disinfection system 1200, such that a single modular unit can be removed while the remaining, assembled modular units remain in place, e.g., supported by one another and/or surrounding support structure (e.g., support frame 1238). Placement of a new modular unit (or the old modular unit after undergoing inspection and/or repair) can then be efficiently accomplished without requiring significant reassembly efforts.

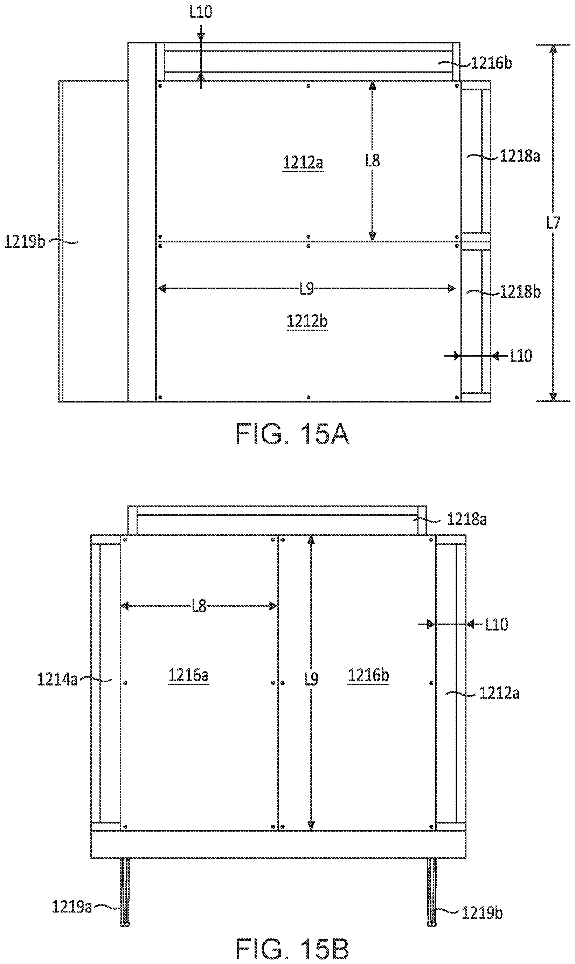

FIG. 15A depicts a side view of disinfection system 1200, and FIG. 15B depicts a top view of disinfection system 1200. As identified in FIGS. 15A and 15B, each modular unit 1212a, 1212b, 1214a, 1214b, 1216a, 1216b, 1218a, 1218b can have a width L8, a length L9, and a thickness L10. Two modular units can be stacked, one on top of the other, to form each of side walls 1212, 1214 and back wall 1218, and two modular units placed side-by-side can form top wall 1216. Accordingly, the length L9 of the modular units can be equal to two times the width L8 of the modular units.

According to some embodiments, disinfection system 1200 can be sized to receive medical equipment 1280, such as wheelchairs, IV poles, medical carts, mobile or portable computer stations, dialysis machines, anesthesia machines, ECG machines, endoscopy equipment, surgical equipment, diagnostic equipment, and/or other types of mobile medical equipment. To form a chamber 1224 and an opening 1230 that are sized to receive standard-sized wheelchairs, portable computer stations, and/or medical carts, modular units having a width L8 of approximately 635mm (25 inches) and a length L9 of approximately 1270mm (50 inches) can be used. Two modular units can be stacked on each of three sides of disinfection system 1200 (i.e., walls 1212, 1214 and back wall 1218) and two modular units can be used for top wall 1216, such that a 1270-by-1270-by-1270mm (50-by-50-by-50-inch) enclosure can be formed.

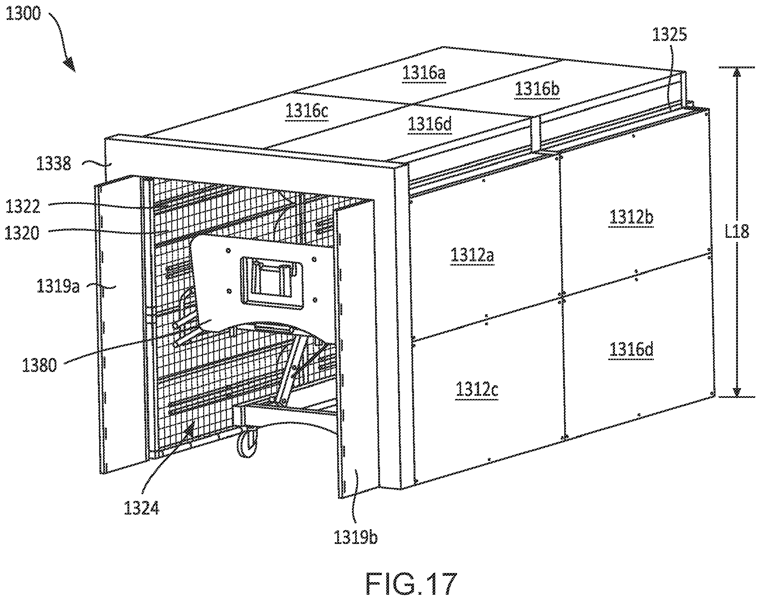

Alternatively, modular units having a width L8 of approximately 635mm (25 inches) and a length L9 of approximately 1270mm (50 inches) can be arranged two-by-two to form two side walls, two high to form a back wall, and two-by-two to form a top wall, such that a 1270-by-2540-by-1270mm (50-by-100-by-50-inch) enclosure can be formed. Examples of medical equipment that can be received in such an enclosure can include standard-sized gumeys, wheelchairs, portable computer stations, and/or medical carts. FIG. 17 depicts an example disinfection system 1300 having such an arrangement. As depicted, each of walls 1312, 1314 can be formed of four modular units (i.e., wall 1312 can be formed of modular units 1312a, 1312b, 1312c, 1312d, and wall 1314 can be formed of modular units 1314a, 1314b, 1314c, 1314d), back wall (not depicted) can be formed of two modular units, and a top wall 1316 can be formed of four modular units 1316a, 1316b, 1316c, 1316d. Disinfection system 1300 can be similar to disinfection system 1200, except for the different arrangement of modular units. Accordingly, disinfection system 1300 can have panel sections 1319a, 1319b, a chamber 1324, reflective units 1320, energy sources 1322, connectors 1325, and a support frame 1338, similar to those of disinfection system 1200. As depicted, disinfection system 1300 can be sized to receive a piece of medical equipment, such as a gurney 1380, within its chamber 1324.

Panel sections 1319a, 1319b can function as a door of disinfection system 1300. Panel sections 1319a, 1319b can each be bi-folding doors that fold to expose an opening to chamber 1324 and unfold to close the opening. While two panel sections or bi-folding doors are shown in FIG. 17, it can be appreciated that a single larger bi-folding door that is attached to one side of support frame 1338 can be used in the alternative. Panel sections 1319a, 1319b can be structurally and/or functionally similar to panels sections 1219a, 1219b, and disinfection system 1300 can be configured to operate panel sections 1319a, 1319b similar to panels sections 1219a, 1219b. For example, disinfection system 1300 can be configured to automatically open and/or close panel sections 1319a, 1319b, e.g., in response to a user identifying himself via a badge or other identification device.

Alternatively, modular units having a width L8 of approximately 635mm (25 inches) and a length L9 of approximately 50 inches can be arranged three high to form three sides (i.e., two side walls and a back wall) and two long to form a top wall, such that a 1270-by-1270-by-1905mm (50-by-50-by-75-inch) enclosure can be formed. Examples of medical equipment that can be received in such an enclosure can include standard-sized wheelchairs, wheelchairs with attached IV poles, IV poles, portable computer stations, medical carts, dialysis machines, and/or anesthesia machines.

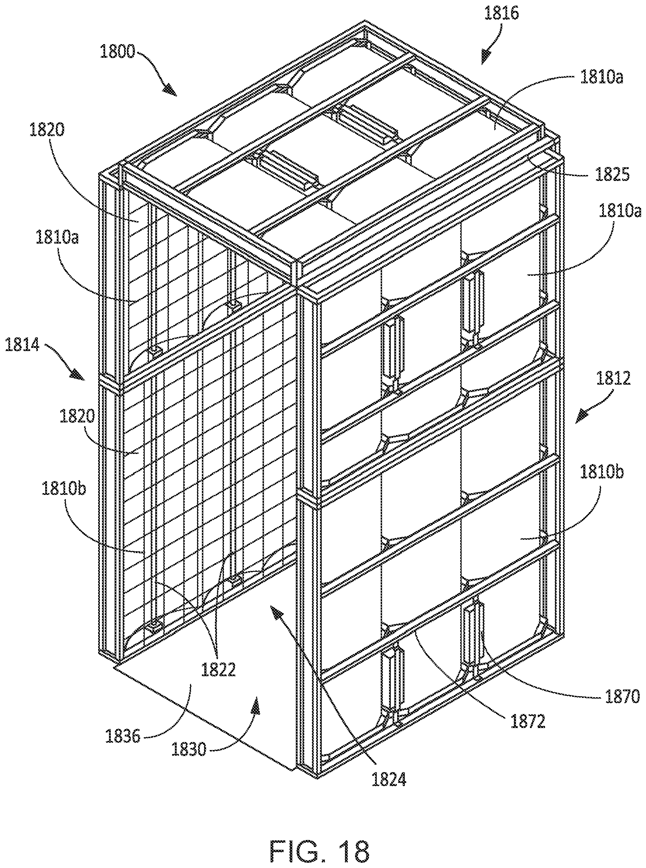

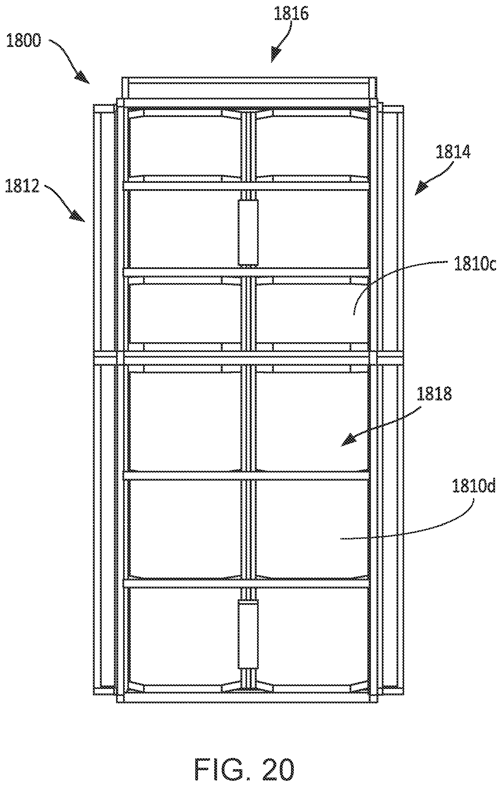

FIGS. 18-20 illustrate different views of an example disinfection system 1800. Disinfection system 1800 can be similar to other disinfection systems described herein (e.g., disinfection systems 100, 200, 700, 800, 900, and/or 1200), and can include components that are structurally and/or functionally similar to the components of those systems. For example, disinfection system 1800 includes a plurality of walls 1812, 1814, 1816, 1818 that define a chamber 1824. Each wall 1812, 1814, 1816, 1818 can be formed of one or more modular units.

Depending on the size requirements of walls 1812, 1814, 1816, 1818, the modular units used to form those walls can have specific dimensions and/or configurations. For example, wall 1812 can be formed of two types of modular units, e.g., a first type of modular unit 181 0a and a second type of modular unit 181 0b. Wall 1814 can be formed of the same two types of modular units. Wall 1816 can be formed of a single modular unit, e.g., the first type of modular unit 1810a. And wall 1818 can be formed of two types of modular units, e.g., a third type of modular unit 1810c and a fourth type of modular unit 1810d. Altogether, walls 1812, 1814, 1816, 1818 can be formed of four different types of modular units 1810a, 1810b, 1810c, 1810d. While four different types of modular units are depicted in FIG. 18, it can be appreciated that any number of types of modular units can be used to form disinfection systems described herein.

Each of the modular units 1810a, 1810b, 1810c, 1810d can include a set of reflective units or surfaces 1820 and a set of energy sources 1822. Reflective units 1820 and/or energy sources 1222 can be similar to other reflective units and energy sources described herein. For example, each reflective unit 1820 can have a reflective surface that can reflect energy emitted by energy sources 1822 into chamber 1824. As depicted in FIGS. 18-21D, each reflective unit 1820 can have a hyperbolically shaped reflective surface that can be configured to distribute energy emitted by energy sources 1822 and reflect it into chamber 1824. Each energy source 1822 can be disposed within a reflective unit 1820. Energy sources 1822 can include at least one light tube configured to emit disinfecting light (e.g., UV light). Energy sources 1822 can be connected to electrical connectors 1826 disposed within or adjacent to each reflective unit 1820. While not depicted, it can be appreciated that additional reflective units and/or energy sources can be disposed on additional walls or surfaces of disinfection system 1800.

The four types of modular units 1810a, 1810b, 1810c, 1810d can have different dimensions and/or configurations. Each modular unit 1810a, 1810b, 1810c, 1810d can be designed to couple to adjacent modular units via one or more connectors 1825. For example, connectors 1825 can include snap-on components that can mate with one another to connect two modular units together. Alternatively or additionally, connectors 1825 can include fasteners, adhesives, magnets, etc. that can adhere two adjacent modular units to one another. In some embodiments, connectors 1825 can include electrical connections and/or fluid connections, which can connect to electrical connections and/or fluid connections in adjacent modular units such that a network of electrical connections and/or fluid connections can be formed to connect one or more components of modular units 1810a, 1810b, 1810c, 1810d to power source(s), fluid source(s), a control panel, a processor, and/or other centralized elements.

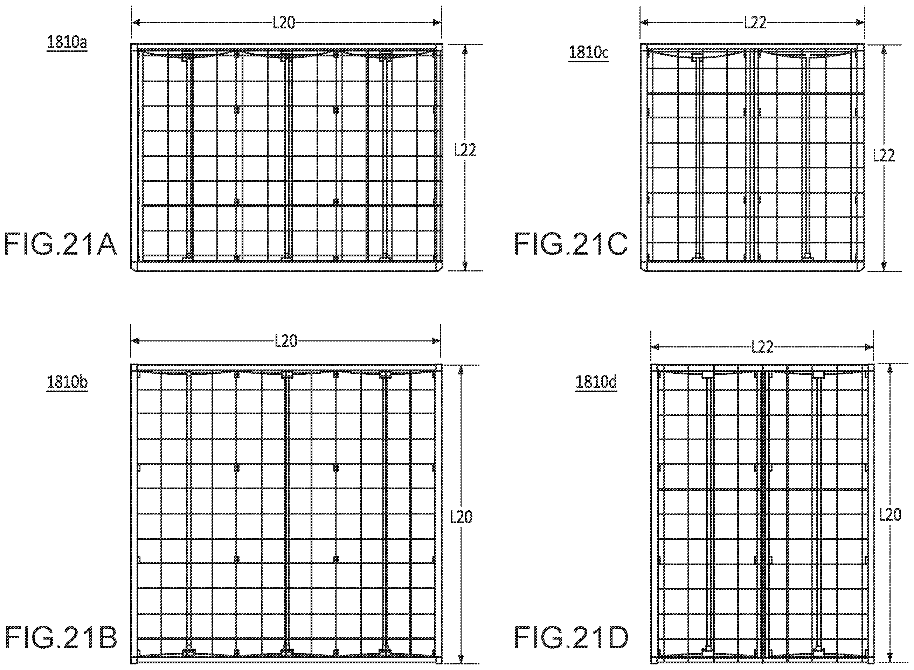

The modular units 1 810a, 181 0b, 1810c, 1810d are dimensioned such that they fit with one another to form a box-shaped disinfection system 1800. For example, as shown in FIGS. 21A-21D, modular unit 1810a can be L20-by-L22, modular unit 1810b can be L20-by-L20, modular unit 1810c can be L22-by-L22, and modular unit 1810d can be L22-by-L20, such that the modular units 1810a, 1810b, 1810c, 1810d form a chamber 1824 having a length of approximately L20, a width of approximately L22, and a height of approximately L20 and L22. In an embodiment, L20 and L22 can be less than about 1270mm (50 inches). In an embodiment, L20 can be approximately 1219.2mm (48 inches) and L22 can be approximately 914.4mm (36 inches). In an embodiment, L20 and L22 are each multiples of a common value (e.g., 304.8mm or 12 inches), and are at least equal to or greater than that common value and less than approximately four times that common value. In an embodiment, L20 and L22 can each be at least between approximately 304.8mm (12 inches) and approximately 1219.2 mm (48 inches). In an embodiment, L20 and L22 can be equal to one another.

While disinfection system 1800 is depicted as being box-shaped (e.g., having a rectangular cross-section), it can be appreciated that other shapes and/or configurations of disinfection system 1800 can be used, e.g., for receiving different sized objections as further described herein.

When disinfection system 1800 forms a chamber 1824 having dimensions of approximately 914.4-by-1219.2-by-2133.6mm (36-by-48-by-84 inches) (e.g., when L20 is approximately 1219.2mm (48 inches) and L22 s approximately 914.4mm (36 inches)) with an opening 1830 of approximately 914.4-by-2133.6mm (36-by-84 inches), disinfection system 1800 can be sized to receive medical equipment, such as, for example, wheelchairs, IV poles, medical carts, computer stations, dialysis machines, anesthesia machines, ECG machines, etc. Alternatively, different arrangements and/or types of modular units can be used to accommodate other types of medical equipment. For example, multiple 914.4-by-914.4mm (36-by-36 inch) modular units (e.g., modular unit 1810c) can be used to form a smaller enclosure that is 914.4-by-914.4-by-914.4mm (36-by-36-by-36 inches), which can be used to disinfect wheelchairs without IV poles, computer stations, etc. As another example, two 1219.2-by-1219.2mm (48-by-48 inch) modular units can be arranged side-by-side to form the side and top walls of a disinfection unit such that a longer chamber of approximately 96 inches in length can be formed to receive longer equipment such as gurneys.

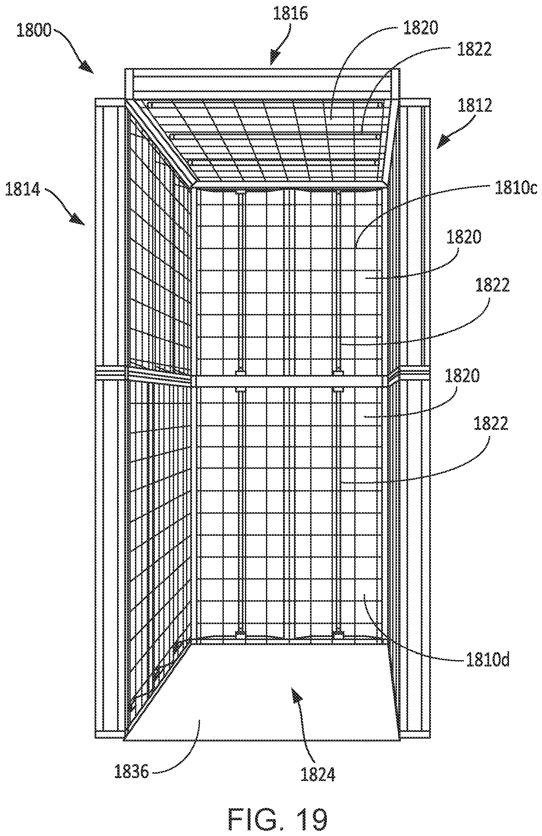

Disinfection system 1800 can include a bottom wall 1836, as depicted in FIGS. 18 and 19. Bottom wall 1836 can be formed of a signal unitary piece or be formed of multiple pieces that couple together to form a generally flat bottom surface for supporting objects within chamber 1824. The bottom wall 1836 can have a reflective surface (e.g., for further reflecting and directing light energy toward an object being disinfected) or non-reflective surface. In an embodiment, bottom wall 1836 can be a rigid and durable material, e.g., a metal such as stainless steel. The bottom wall 1836 of the disinfection system 1800 can prevent energy and/or disinfecting agent(s) being delivered within chamber 1824 from affecting (e.g., degrading, discoloring) a floor or other surface upon which the disinfection system 1800 is positioned.

Disinfection system 1800 can optionally include a front door or wall (not depicted). For example, similar to other disinfection systems described here, disinfection system 1800 can include a door that is hinged, rolling, folded (e.g., bi-folded), sliding, etc. The front door can be movable between a closed position and an open position. In the closed position, the front door can prevent energy and/or disinfection agent(s) within the chamber 1824 from exiting the chamber and affecting surrounding objects and/or persons. In the open position, the front door can enable an object to be positioned within and/or removed from the chamber 1824.

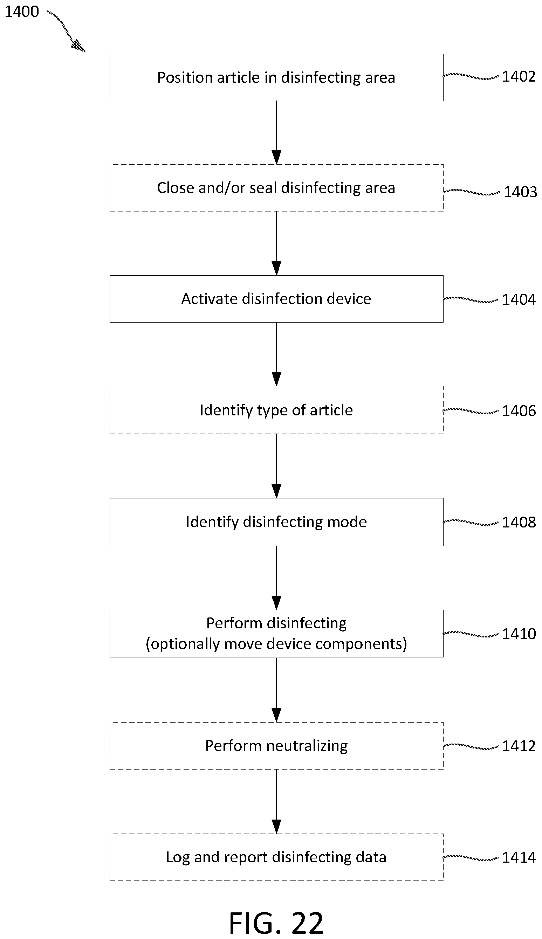

FIG. 22 is a flow chart of a method 1400 for disinfecting using a disinfection device, such as any of the disinfection systems disclosed herein (e.g., disinfection systems 100, 200, 700, 800, 900, 1200, 1300, 1700, 1800, 2600). An object or article (e.g., a piece of medical equipment) can be placed in a disinfecting area, at 1402. Disinfecting area can be an open space adjacent to a disinfection device and/or a space within a chamber (e.g., chamber 124, 724, 824, 924, 1224, and/or 1334) defined by a disinfection device. Optionally, the disinfecting area can be closed or sealed, at 1403. The disinfection device can be activated, at 1404. For example, a user can use his badge to turn on and activate the disinfection device. Alternatively, a user located at a remote location can use a control panel to activate the disinfection device. Optionally, at 1406, the disinfection device, e.g., via sensors (e.g., sensor(s) 164) and/or user inputs via a control panel having a user interface (e.g., control panel 150 having I/O interface 152), can identify the type of article, e.g., the type of medical equipment. Based on the type of article that is identified, or based on other information in putted by a user (e.g., via the control panel and/or user interface), the disinfection device can identify a disinfecting mode to use, at 1408.

At 1410, the disinfection device can perform the disinfecting according to the disinfecting mode. In some embodiments, the disinfection mode may involve disinfecting using energy (e.g., UV light and/or pulses of HINS light) and one or more disinfecting agents (e.g., disinfecting agent 190). For example, a set of energy sources capable of emitting energy at an intensity capable of disinfecting a surface of the article can be energized, and at least one disinfecting agent can be delivered to the disinfecting area via a set of spray units.

One or a combination of disinfecting agents may be used: aerosolized or vaporized hydrogen peroxide, aerosolized or vaporized peracetic acid-hydrogen peroxide combination, aerosolized or vaporized electrolyzed water, aerosolized or vaporized cold atmospheric pressure plasma, or aerosolized or vaporized polymeric guanidine. Optionally, prior to and/or during the disinfection process, the disinfection device can move device components (e.g., reflective units, power sources, spray units, exhaust units, sensors, etc.) to perform the disinfection. For example, the disinfection device can move device components to position certain components closer to the article being disinfected, e.g., to increase efficiency and/or efficacy of disinfection. Optionally, at 1412, the disinfection device can perform neutralizing by applying a neutralizing agent (e.g., neutralizing agent 192), to reduce residual disinfecting from degrading the surfaces of the article being disinfecting and/or to reduce the risk of harmful contact of the disinfecting agent with a human after the article is removed from the disinfecting area. Optionally, at 1414, the disinfection device, via sensors, processors, communication channels, etc., can log and/or report disinfecting data, such as, for example, the article that was disinfected, the user initiating the disinfection, etc.

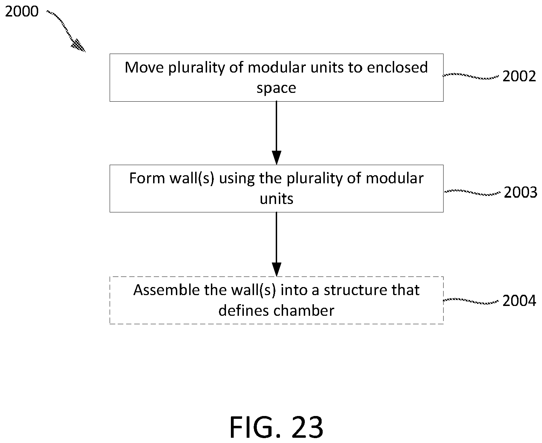

FIG. 23 depicts a flow chart of a method 2000 for assembling a disinfection device, such as any of the disinfection systems disclosed herein (e.g., disinfection systems 100, 200, 700, 800, 900, 1200, 1300, 1700, 1800, 2600). A plurality of modular units can be moved from a first location outside of an enclosed space (e.g., a room of a hospital) to a second location inside the enclosed space though an opening (e.g., a doorway), at 2002. Each modular unit can be sized to fit through the opening. The modular units can be assembled into one or more walls or panels, at 2003. Optionally, at 2004, the modular units can be assembled into one or more walls forming a structure that defines a chamber sized to receive an object (e.g., a piece of medical equipment). In some embodiments, the modular units can first be assembled into a plurality of walls, and the plurality of walls can be arranged to form the structure that defines the chamber.

FIG. 24 is a flow chart of a method 2100 for disinfecting using a disinfection system (e.g., any of the disinfection systems described herein, such as, for example, disinfection systems 100, 200, 700, 800, 900, 1200, 1300, 1700, 1800, 2600), according to embodiments described herein. A disinfection system can include, for example, one or more sensor(s), processor(s), and/or input/output (I/O) devices, which enable the system to obtain information regarding users using a disinfection system and/or objects being disinfected, and to control disinfection based on such information. In some embodiments, disinfection systems described here can be designed to increase safety, e.g., by reducing or eliminating a risk of exposure to UV-C or ultraviolet germicidal irradiation (UVGI) to users and other individuals nearby a disinfection system. At 2102, the disinfection system can optionally read user identification information, e.g., information from a badge of a user. At 2103, the disinfection system can optionally determine whether the user is permitted to perform disinfection, e.g., by authenticating the user. In some embodiments, the user can be authenticated based on information obtained from a badge or other identification of a user, e.g., using a RFID sensor or other type of near field communication (NFC) device. In some embodiments, the user can be authenticated based on information provided by a user into a control panel or other input device that is operatively coupled to the disinfection system (e.g., control panel 150, a compute device (e.g., mobile device) connected to the disinfection system, etc.). When the user is authenticated (2103: YES), the process can proceed onwards, e.g., to performing the disinfection. When the user cannot be authenticated (2103: NO), the disinfection system can optionally present or communicate an alert or error to the user, e.g., to inform the user (or another user, e.g., an administrator) that the user was not authenticated, at 2116. For example, the disinfection system can present on an onboard display that the user cannot be authenticated, or the disinfection system can send a message to a remote compute device (e.g., one associated with an administrator) to alert an administrator or other users that an unauthorized user had attempted to use the disinfection system. In some embodiments, the disinfection system can be configured to provide access to an interior of the disinfection system (e.g., a chamber 124 or disinfecting area 125) to users that have been authenticated and authorized to operate the disinfection system (e.g., trained users) and not any other users. For example, the disinfection system can include a door that is closed when not in use. In response to a user authenticating himself, e.g., the disinfection system determining that the user is authorized the use the disinfection system (2103: YES), the disinfection system can be configured to automatically open the door or the user can be allowed to open the door. Then after placing an object within the interior of the disinfection system, the disinfection system can cause the door to automatically close or the user can be instructed to close the door.

At 2104, the disinfection system can optionally detect whether an object has been placed in the disinfection area (e.g., disinfecting area 124). For example, the disinfection system using one or more of its sensor(s) (e.g., sensor(s) 164) can determine whether an object is disposed within its chamber and/or near its reflective units and energy sources, such that the object can be disinfected. In some embodiments, if one or more objects are detected, the disinfection system can cause a door providing access to the disinfecting area to automatically close. In some embodiments, if no object is detected after a predefined period of time, the disinfection system can alert the user and/or automatically close the door to the disinfection area, e.g., to prevent unauthorized use of the disinfection system.

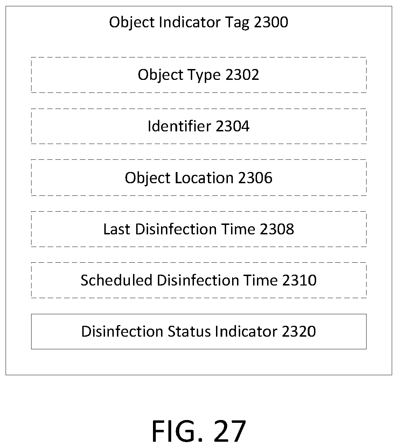

At 2105, the disinfection system can optionally read an indicator tag of the object. The indicator tag can be a digital tag, e.g., a NFC tag such as a RFID tag or Bluetooth tag. In some embodiments, the indicator tag can present information visually (e.g., on an viewable surface). In other embodiments, the NFC tag can store information on an onboard memory, which can be read by a NFC device. FIG. 27 provides an example of an object indicator tag 2300. Object indicator tag 2300 can include information such as a type 2302 of the object (e.g., wheelchair, IV pole, gurney, etc.), an identifier 2304 of the object (e.g., a barcode, an unique identifier, etc.), a location 2306 of the object (e.g., a room or a floor that the object should be located in), a last disinfection time 2308 of the object (e.g., a past calendar date), a scheduled disinfection time 2310 of the object (e.g., a future calendar date), or a disinfection status indicator 2320. The disinfection status indicator can indicate whether the object associated with the tag has been disinfected according to protocol or has not been.

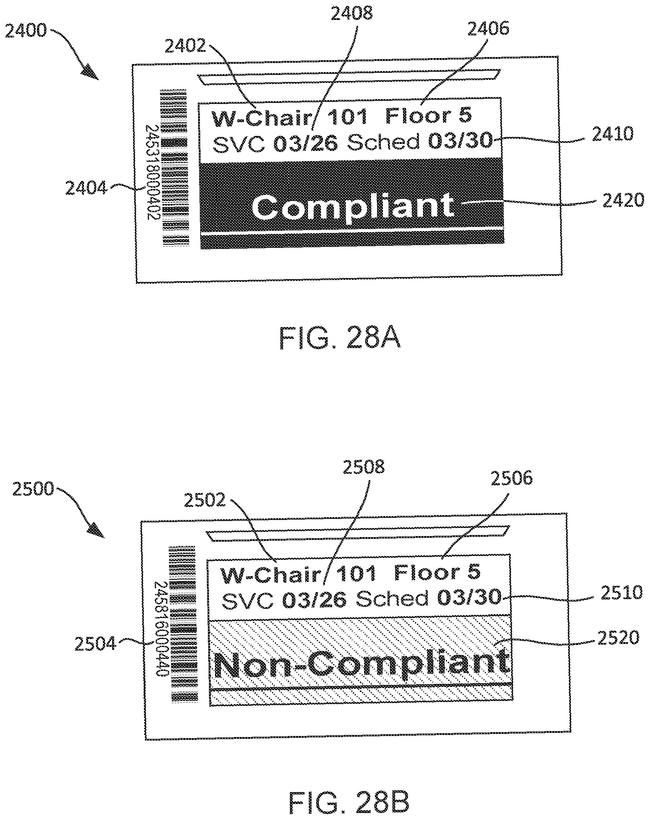

FIGS. 28A and 28B provide specific examples of disinfection or indicator tags 2400, 2500 that include visual surfaces that present information, such as the information depicted in FIG. 27. Disinfection tags 2400, 2500 can be disinfection tags of a wheelchair. Indicator tags 2400, 2500 can include identifiers 2404, 2504, respectively. Indicator tags 2400, 2500 can include information indicating a type 2402, 2502 of the object, i.e., wheelchair. Indicator tags 2400, 2500 can include a location 2406, 2506 of the object, i.e., a floor (Floor 5) that the object should be located on. Indicator tags 2400, 2500 can indicate a last disinfection time 2408, 2508 of the object, as well as a scheduled disinfection time 2410, 2510 of the object. Indicator tags 2400, 2500 can be programmed to indicate whether the object is compliant or non-compliant with disinfection protocols, e.g., using status indicators 2420, 2520. For example, indicator tag 2400 can include status indicator 2420 that has been programmed to be "Compliant" and colored a first color (e.g., black) to indicate that the object associated with the indicator tag 2400 is compliant with disinfection protocols. Indicator tag 2500 can include status indicator 2520 that has been programmed to be "Non-Compliant" and colored a second color (e.g., red) to indicate that the object associated with the indicator tag 2500 is non-compliant with disinfection protocols. In some embodiments, when an object is non-compliant or becomes non-compliant, the indicator tag can be configured to change color and/or change a marking to signify to users and/or compute devices (e.g., a disinfection system or monitoring system) that the object is non-compliant and therefore needs to be disinfected. Such can facilitate in identifying objects that are non-compliant and encourage the disinfection of such objects.

In some embodiments, the indicator tags 2400, 2500 can be hermetically sealed (e.g., weatherproof and waterproof). In some embodiments, the indicator tags 2400, 2500 can have a non-luminous screen. In some embodiments, the indicator tags 2400, 2500 can be configured to not generate any noise such that a patient environment is not disrupted during use of the indicator tags 2400, 2500.

Referring back to the flow depicted in FIG. 24, at 2106, the disinfection system can optionally receive a user input. For example, a user can enter information into the disinfection system instructing the disinfection system to perform a disinfection, including information indicating a type or mode of disinfection, information about the object (e.g., information such as object type, object size, etc.), information about the user, etc. At 2108, the disinfection system can determine whether the object requires disinfection. For example, the disinfection system can determine whether the object is in compliance with disinfection protocols. When the object is compliant (2108: NO), then the disinfection system can optionally present or communicate to the user that the object is already compliant with protocols and may not need to be disinfected, at 2116. In such instances, the user can then determine whether the user still wants to disinfect the object and, if so, the user can proceed with the disinfection by providing an input to the disinfection system. When the object is not compliant (2108: YES), the disinfection system can determine that the object requires disinfection . In some embodiments, the disinfection system can be configured to scan the object and determine whether the object includes pathogens or other contaminants and needs to be disinfected. In some embodiments, the disinfection system can receive a user input that indicates to the disinfection system that the object requires disinfection. In some embodiments, the disinfection system can read, e.g., from an object indicator tag, that the object is non-compliant with disinfection protocols and requires disinfection.

At 2109, the disinfection system can determine whether it is safe or ready to perform the disinfection. In some embodiments, the disinfection system can determine that the object has been placed in the disinfection area, and that the disinfection area has been sealed (e.g., the chamber door has been closed). In some embodiments, the disinfection system can determine that there is no movement in the disinfection area before performing the disinfection. In some embodiments, the disinfection system can include a motion sensor, cameras, or other sensors, e.g., to determine whether there is motion or a living being within the disinfection area. In some embodiments, the disinfection system can be configured to determine whether the door to the disinfection system is obstructed, e.g., to ensure that the door does not close on a user or object. In some embodiments, the disinfection system can be configured to ensure that the door does not pinch a user or object when the door is in a closed position or configuration. In some embodiments, the disinfection system can include one or more of a door obstruction sensor or a door pinch sensor, such as, for example, a force sensor, a light sensor, etc. In some embodiments, the disinfection system may wait for a user or prompt a user to provide an input indicating that the system is ready for disinfection before performing the disinfection. Further details of specific safety features are described with reference to example method 3000 depicted in FIG. 30.

When the disinfection system determines that it is not safe or ready to perform the disinfection (2109: NO), the disinfection system can optionally present or communicate this to the user or an administrator, at 2116. In some embodiments, the disinfection system can communicate to the user the reason that the system is not safe or ready and/or provide instructions to the user on how to make the disinfection system safe or ready for use. When the disinfection system determines that it is safe and ready for disinfection (2109: YES), then the disinfection system can disinfect the object, at 2110. Disinfecting the object can include one or more of activating the disinfection system (e.g., similar to 1404 as depicted and described with reference to FIG. 22), identifying a type of the object (e.g., similar to 1406 as depicted and described with reference to FIG. 22), identifying a disinfecting mode (e.g., similar to 1408 as depicted and described with reference to FIG. 22), and performing disinfection (e.g., similar to 1410 as depicted and described with reference to FIG. 22).

Upon, during, or after performing the disinfection, the disinfection system can optionally log and report the disinfection, at 2112. For example, the disinfection system can store on a local memory that it had disinfected the object at a particular time and date. Alternatively or additionally, the disinfection system can send information indicating that it had disinfected the object to a remote compute device, such as a server, e.g., via a network, such that the server can store the information centrally with other disinfection information, e.g., disinfection information provided by other disinfection devices. At 2114, the disinfection system can optionally program the object indicator tag to show that the object is compliant with disinfection protocols. For example, the disinfection system can program an object indicator tag to show "Compliant" and to be a certain color indicative of compliance, such as that depicted in FIG. 28A and described above. At 2116, the disinfection system can optionally present or communicate the status of the disinfection to the user, e.g., communicate using an onboard display that the disinfection is complete.

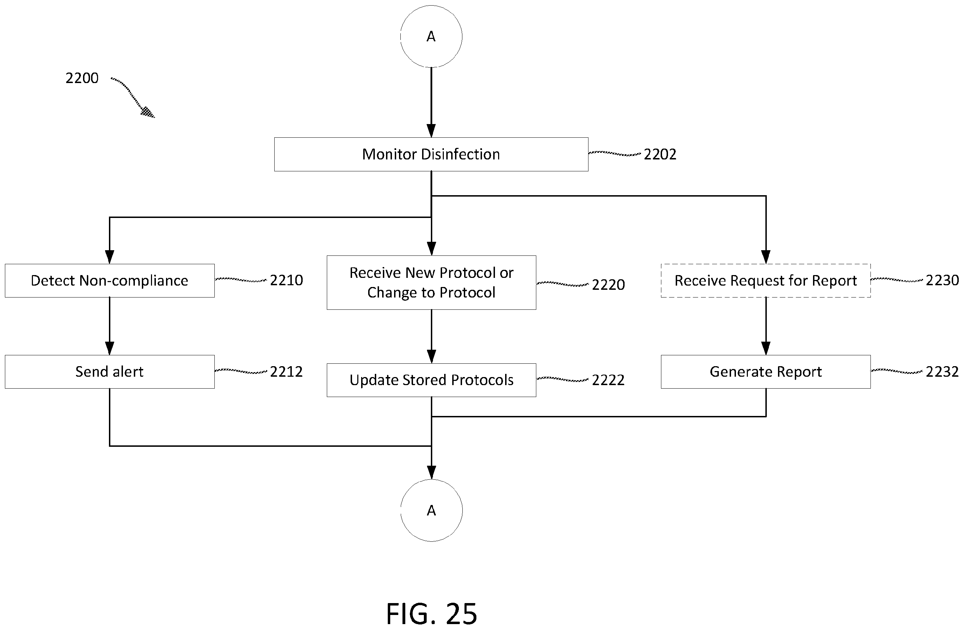

FIG. 25 depicts a flow chart of a method 2200 of monitoring and tracking information about the disinfection. Method 2200 can be performed by a disinfection system, such as any described herein (e.g., disinfection systems 100, 200, 700, 800, 900, 1200, 1300, 1700, 1800, 2600). Alternatively or additionally, method 2200 can be performed by a remote device, e.g., a cloud platform or server, that remotely monitors one or more disinfection systems (e.g., one or more disinfection systems within a hospital or other medical facility) and tracks disinfection information for the collective group of disinfection systems. For example, method 2200 can be performed by a remote device that updates one or more disinfection logs associated with objects being disinfected. Accordingly, method 2200 is described below by making reference to a general compute device, which can be, for example, a processor of a disinfection system or a remote cloud platform or server. At 2202, the compute device can monitor disinfection, e.g., by monitoring information regarding disinfections that are performed by one or more disinfection systems. In some embodiments, the compute device may automatically receive reports from one or more disinfection systems, indicating disinfections that are performed by those systems. In some embodiments, the compute device can request or obtain information regarding disinfections being performed from one or more disinfection systems. At 2210, the compute device can detect non-compliance with a disinfection protocol. For example, the compute device can detect that an object that should have been disinfected at a scheduled time based on a disinfection protocol was not disinfection at that scheduled time. As another example, the compute device can detect that an object as disinfected in error, e.g., when another object should have been disinfected. In response to detecting that there is non-compliance, the compute device can send an alert, e.g., to a user near the disinfection device or a remote administrator, such that they can correct the non-compliance.

At 2220, the compute device can receive a new protocol or a change to an existing protocol. In some embodiments, the compute device can be operatively coupled to a terminal or control panel that a user (e.g., an administrator) can provide new protocols and changes to protocols. In some embodiments, the compute device can receive a message from another compute device (e.g., controller, processor) that may have changed a disinfection protocol, e.g., based on hospital needs, increasing equipment usage, etc. At 2222, the compute device can update its stored protocols based on the received protocols or changes. For example, the compute device can store in a local memory or remote memory that is operatively coupled to the compute device the new protocol or change to the existing protocol. The compute device can then refer to this new protocol or change in protocol when determining whether future disinfections are in compliance with such protocols. In some embodiments, the compute device can synchronize these changes across a plurality of disinfection systems, such that each disinfection system can have an updated copy of the disinfection protocols.

At 2230, the compute device can optionally receive a request for a report of the disinfections being performed. At 2232, the compute device can generate the report, e.g., in response to the request, or at periodic intervals. The report can be, for example, a report that documents disinfection incidents, compliance statistics (number of non-compliances, percentage of overall compliance), disinfection statistics (e.g., number of disinfections performed over time and/or by type of object), etc.

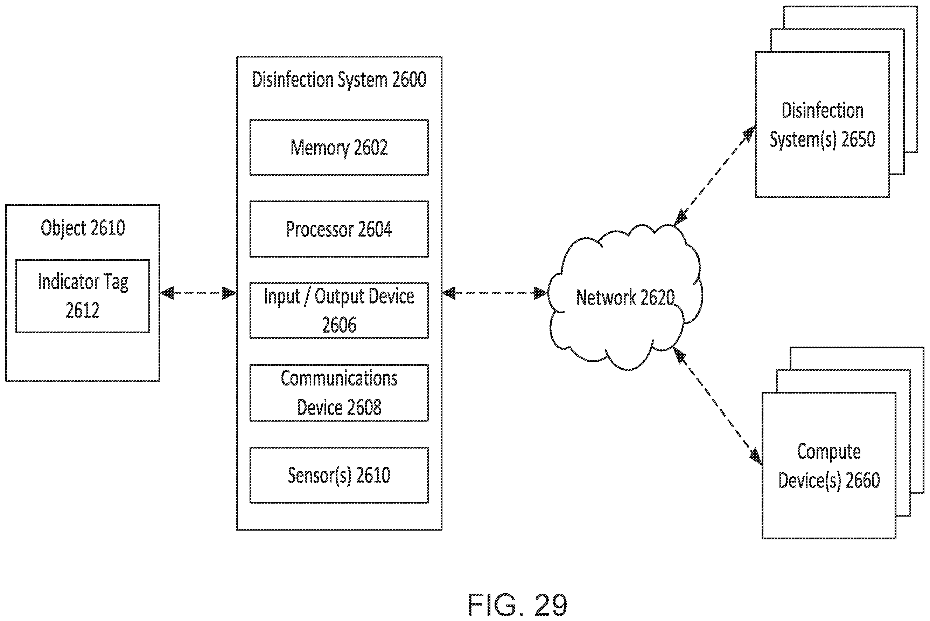

FIG. 29 is a schematic illustration of a network of devices that can communicate with one another and exchange information between themselves and/or remote devices via a network (e.g., remote devices that form part of a cloud platform). Disinfection system 2600 can be connected, e.g., via a communications device 2608, to a network 2620, with which the disinfection system 2600 can communicate with one or more other disinfection system(s) 2650 and/or one or more compute device(s) 2660. For example, disinfection system 2600 and additional disinfection system(s) 2650 can be connected to one another such that they can share information regarding disinfections that they perform with each other. Disinfection system 2600 and additional disinfection system(s) 2650 can also be connected to one or more remote compute device(s) 2660 (e.g., servers, administrator compute devices), and can send information to those devices such that the information can be collected and/or analyzed by those devices. In some embodiments, disinfection system 2600 can be connected to user compute devices, e.g., mobile devices, tablets, laptops, etc., such that the user can be configured to monitor information and/or provide information via their personal devices. In some embodiments, disinfection system 2660 can be configured to track disinfection cycles of one or more objects being disinfected, e.g., by updating one or more logs to reflect a history of disinfection of the one or more objects.

Network 2620 can be any type of network (e.g., a local area network (LAN), a wide area network (WAN), a virtual network, a telecommunications network) implemented as a wired network and/or wireless network and used to operatively couple compute devices, including disinfection systems 2600, 2650 and compute device(s) 2660. As described in further detail herein, in some embodiments, for example, the compute devices are computers connected to each other via an Internet Service Provider (ISP) and the Internet (e.g., network 105). In some embodiments, a connection can be defined, via network 2620, between any two compute devices. In some embodiments, the compute devices can communicate with each other (e.g., send data to and/or receive data from) and with the network 2620 via intermediate networks and/or alternate networks (not shown in FIG. 29). Such intermediate networks and/or alternate networks can be of a same type and/or a different type of network as network 2620. Each compute device can be any type of device configured to send data over the network 2620 to send and/or receive data from one or more of the other compute devices.

Disinfection system 2600 can include components that are structurally and/or functionally similar to other disinfection systems described herein (e.g., disinfection systems 100, 200, 700, 800, 900, 1200, 1300, 1700, 1800, 2600). For example, disinfection system 2600 can include a processor 2604, e.g., similar to processor 154 of disinfection system 100. Disinfection system 2600 can also include an input/output device 2606, e.g., for presenting information to a user and/or receiving inputs from a user. Disinfection system 2600 can include one or more sensor(s) 2610, e.g., similar to sensor(s) 164 of disinfection system 100. Sensor(s) 2610 can be configured to monitor one or more conditions of a disinfection area, one or more characteristics of objects being disinfected, and/or one or more conditions of the disinfection chamber 2600 and/or its surrounding environment. For example, sensor(s) 2610 can monitor disinfections being performed by the disinfection system 2600 (e.g., are objects being disinfected according to disinfection protocols) as well as a state of the disinfection system 2600 (e.g., whether components need to be replaced, etc.). Disinfection system 2600 can have a memory 2602 that can store, for example, disinfection data (e.g., before communicating the data to a remote device, or for certain periods of time). Similar to other disinfection systems described above, disinfection system 2600 can be configured to obtain information from an object 2610, e.g., via an indicator tag 2612 of the object.

In some embodiments, disinfection system 2600 can be configured to log information regarding one or more disinfection cycles (e.g., time and date of a cycle, user or operator that initiated the cycle, object being disinfected, type of disinfection, status of disinfection, errors or alerts during cycle), as described with reference to FIG. 24, and provide that information via network 2620 to one or more remote compute device(s) 2660. The remote compute device 2660 can be, for example, an administrator device or other central device that is used to monitor disinfections across one or more institutions (e.g., a hospital, hospital network, nursing home, grocery store, etc.). In some embodiments, the remote compute device 2660 can be configured to send messages (e.g., electronic communications, push notifications, etc.) to one or more users at these institutions when an object needs to be disinfected and/or an issue needs to be addressed (e.g., motion being in a disinfection unit, a door safety issue, etc.). In some embodiments, disinfection system 2600 can be configured to send information regarding a state of the disinfection system to one or more remote compute device(s) 2660. For example, the disinfection system 2600 including sensor(s) 2610 can be configured to detect with an energy source (e.g., energy source 122), reflective unit (e.g., reflective unit), or other element of the disinfection system 2600 is mal-functioning, due for a maintenance check, or needs to be replaced. The disinfection system 2600 can send this information to a remote compute device 2660 associated with a service or maintenance provider of the disinfection system 2600 and/or an administrator device, such that the remote compute device 2660 can schedule a maintenance or repair of the disinfection system 2600. In some embodiments, a remote compute device 2660 can be configured to remotely monitor a status of the disinfection system, e.g., by turning on one or more sensors (e.g., cameras, light sensors, force sensors, temperature sensors) to inspect the disinfection unit. If the remote compute device 2660 detect that there is an issue with the disinfection system 2600, the remote compute device 2660 can alert a user of the issue such that the issue is addressed.

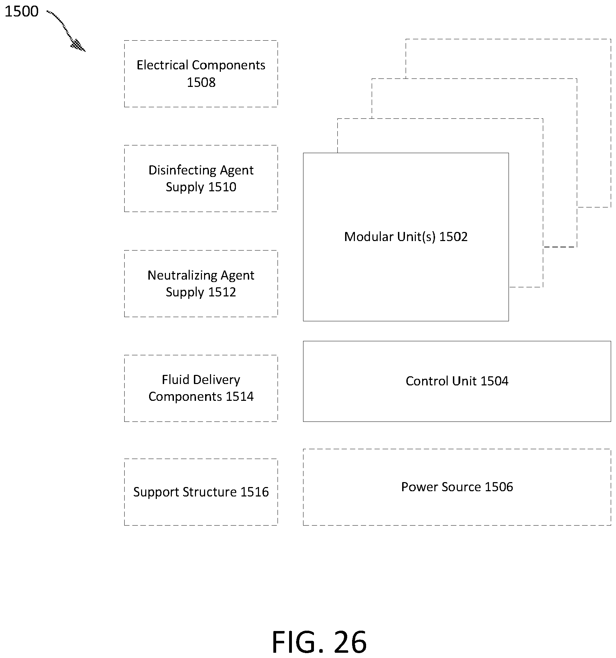

FIG. 26 depicts various components that can form an example disinfection system 1500. Disinfection system 1500 can be structurally and/or functionally similar to any of the other disinfection systems described herein (e.g., disinfection systems 100, 200, 700, 800, 900, 1200, 1300, 1700, 1800, 2600). The components depicted in FIG. 26 can be provided in a kit, which can be delivered to an onsite location (e.g., a room in a hospital) for assembly at the onsite location. As shown, the components can include one or more modular unit(s) 1502. Modular unit(s) 1502 can be similar to any of the other modular units disclosed herein, and can include similar components as those modular units. The components can include a control unit 1504, which can be used to control and operate disinfection system 1500, once it is assembled for use. Control unit 1504 can include, for example, a processor (e.g., processor 154) and/or a control panel (e.g., control panel 150). The components can optionally include a power source 1506 and/or electrical component 1508 connecting other components of disinfection system 1500 to the power source 1506 (e.g., modular unit(s) 1502 and/or components included on modular unit(s) 1502, such as energy sources, reflective units, spray units, exhaust units, sensors, etc.). Alternatively, the components provided in the kit do not include a power source, but they include suitable electrical components to connect one or more components of disinfection system 1500 to a remote power source (e.g., via a power port). Optionally, the component can also include a disinfecting agent supply 1510, a neutralizing agent supply 1512, and/or fluid delivery components 1514 for establishing fluid communication between disinfecting agent supply 1510 and/or neutralizing agent supply 1512 to one or more spray units disposed on modular unit(s) 1502. Optionally, the components can also include support structure 1516, for supporting modular unit(s) 1502 in a specific arrangement and/or coupling modular unit(s) 1502 to one another in a specific arrangement.

In some embodiments, disinfection systems described herein (e.g., disinfection systems 100, 200, 700, 800, 900, 1200, 1300, 1700, 1800, 2600) can include one or more components that are designed to increase a safety of the system. UV-C or ultraviolet germicidal irradiation (UVGI) can cause eye damage and burn a user's skin. As such, it is desirable to reduce or eliminate a risk of exposure to such radiation to operators and other individuals near the disinfection system. In some embodiments, a disinfection system can include a processor (e.g., processor 154) that is configured to provide access to a disinfecting area (e.g., disinfecting area 125) to authorized users (e.g., trained users) and not to any other users. For example, a trained user can use a badge or other identifying device to open a door of the disinfection chamber (e.g., by swiping the badge), and the user can use that same badge to close the door and initiate a disinfection system. The badge or other identifying device of the user can be remotely activated and/or deactivated, e.g., to further increase safety. In some embodiments, a disinfection system can include one or more components (e.g., sensor, processor, safety release mechanisms, etc.) that are configured to detect that a door to the disinfection system is locked before beginning a disinfection procedure, determine that there is no motion or living being in a disinfecting area before beginning a disinfection procedure, ensure that the door to the disinfection system does not close on or pinch a user or other individual, etc.

FIG. 30 depicts an example method 3000 of an operation of a disinfection system, such as any of the disinfection systems described herein (e.g., disinfection systems 100, 200, 700, 800, 900, 1200, 1300, 1700, 1800, 2600), where the disinfection system includes one or more safety features. A disinfection system can include, for example, one or more sensor(s), processor(s), and/or input/output (I/O) devices, which enable the system to obtain information regarding one or more operational or environmental conditions (e.g., information regarding users using a disinfection system, conditions near the disinfection system, and/or conditions or states of objects being disinfected), and to control disinfection based on such information.

The disinfection system can read user identification information, e.g., information from a badge or other identifying device of a user, at 3002. In some embodiments, the disinfection system can include a touchscreen that has a RFID reader (or other NFC reader) that can read a badge of a user. The badge can be remotely activated and/or deactivated, such that a remote administrator (e.g., a hospital administrator) can remotely change the access credentials of a user. When a user scans his badge at the disinfection system, information such as a name of the user and the time and date can be logged by the disinfection system and communicated (e.g., via communications device 2608 and network 2620) to one or more remote devices (e.g., a compute device 2660 such as a server or database).

The disinfection system can determine based on the user identification information and/or other information whether a user is authorized to use the disinfection system, at 3003. For example, the disinfection system can receive the user identification information (e.g., from the user's badge) and determine whether the user is on a list of authorized users. In some embodiments, the disinfection system can be configured to communicate (e.g., via communications device 2608 and network 2620) with a remote compute device (e.g., a compute device 2660 such as a server or database) to obtain a list of authorized users and/or send along the user identification information for the remote compute device to authenticate the user. In some embodiments, the disinfection system can have a list of authorized users stored in an onboard memory (e.g., memory 2602 or another local storage device). In some embodiments, the disinfection system can receive one or more inputs from a user, e.g., via an I/O interface (e.g., I/O interface 152 and/or I/O device 2606), that the disinfection system uses to authenticate the user. For example, a user can be provided a one-time code or other information (e.g., on a user device, such as a mobile device), and the user can be prompted by the disinfection system (e.g., via touchscreen) to input the one-time code.

When the user cannot be authenticated (3003: NO), the disinfection system can optionally present or communicate an alert or error to the user, e.g., to inform the user (or another user, e.g., an administrator) that the user was not authenticated, at 3016. For example, the disinfection system can present on an onboard display that the user cannot be authenticated, or the disinfection system can send a message to a remote compute device (e.g., one associated with an administrator) to alert an administrator or other users that an unauthorized user had attempted to use the disinfection system.

When the user is authenticated (3003: YES), the door of the disinfection system can be opened to provide access to an inner disinfecting area (e.g., disinfecting area 125), at 3004. The disinfection system, when not in use, can remain in a locked state, e.g., with its door in a closed configuration and being locked, such that unauthorized use of the disinfection system is prevented. In some embodiments, the disinfection system can include motors and other electronic components that automatically open the door to the disinfecting area in response to authenticating the user, e.g., such that the user or other individual does not need to touch any portion of the disinfection system to use the disinfection system. More specifically, in response to a user being authenticated after scanning his badge at the disinfection system, the disinfection system (e.g., as controlled by a processor such as, for example, processor 154, 2604) can automatically open the door to the disinfecting area such that the user can move an object into the disinfecting area for disinfection. By enabling the user to gain access to the disinfecting area without having to contact and/or manipulate a door of the disinfection system can help reduce transmission of pathogens or other agents between users that use the disinfection system. In some embodiments, the user can press a button (e.g., on a touchscreen) or provide some other input to the disinfection system, and the disinfection system can be configured to open the door to the disinfecting area.

At 3005, the disinfection system can detect whether an object has been placed in the disinfecting area. For example, the disinfection system using one or more of its sensor(s) (e.g., sensor(s) 164) can determine whether an object is disposed within its chamber and/or near its reflective units and energy sources, such that the object can be disinfected. In some embodiments, the disinfection system can read an indicator tag of an object (e.g., a digital tag such as a NFC tag, RFID tag, or Bluetooth tag), as described above with reference to method 2100 at 2105.

When an object is not detected within a predefined period of time (3005: NO), the disinfection system can optionally close the door to the disinfecting area, at 3006, and optionally present or communicate this to the user or an administrator, at 3016. In some embodiments, the disinfection system can be configured to present a user with one or more messages, e.g., to alert the user, prior to closing the door of the disinfection system. For example, the disinfection system can be configured to present a message to the user (e.g., via a touchscreen or other I/O device) after a first predefined period of time has elapsed that informs the user that no object has been detected. Then, after a second predefined period of time has elapsed, the disinfection system can be configured to automatically close the door to the disinfection chamber, e.g., to prevent unauthorized use of the disinfection chamber. In some embodiments, the disinfection system can a message to the user after closing the door that informs the user that the door has been closed and requests that the user re-scan his badge to gain access to the disinfection system.

When an object is detected within the disinfection system (3005: YES), then the disinfection system can perform one or more safety checks prior to initiating a disinfection procedure. In some embodiments, the disinfection system can be configured to continuously monitor for safety issues, e.g., both before, during, and after a disinfection procedure, and to send alerts to a user or administrator in response to detecting a safety issue. Alternatively, the disinfection system can be configured to detect safety issues prior to and during a disinfection procedure, and can be in an idle state while not in use. As described above, the disinfection system can include one or more sensors (e.g., sensor(s) 164) that the disinfection system can use to monitor for certain conditions that can present a safety concern.

At 3007, the disinfection system can determine whether there is motion and/or living being near or within the disinfecting area. For example, the disinfection system can include one or more motion sensors (e.g., thermal sensor, infrared sensor, light sensor, force sensor, etc.) that can detect when motion is in the disinfecting area. Alternatively or additionally, the disinfection system can include one or more cameras that can capture a view of the disinfecting area (e.g., a real-time view of the disinfecting area), and the disinfection system can present this view (e.g., via a touchscreen or other I/O device) to the user. The user can then view the disinfecting area and monitor for issues, e.g., movement and/or a living being, within the disinfecting area. In some embodiments, the disinfection system can receive an input from a user to pause the disinfection, e.g., when the user sees on the camera view that a living being is within or near the disinfecting area. When motion or a living being is detected (3007: YES), then the disinfection system can present or communicate this to the user or an administrator, at 3016. In some embodiments, the disinfection system can communicate to the user the reason that the system is not safe or ready (e.g., that there is motion within the disinfecting area) and/or provide instructions to the user on how to make the disinfection system safe or ready for use.

At 3009, the disinfection system can determine whether there are one or more door safety issues. For example, the disinfection system can include a sensor that is configured to detect whether the door to the disinfecting area is obstructed, such as a light sensor, force sensor, temperature sensor, etc. positioned near or along a door of the disinfection system. Alternatively or additionally, the disinfection system can include a sensor that is configured to determine whether the door, when closing, would pinch or cause injury to a person or an object, such as, for example, a force sensor that is configure to stop movement of the door and/or re-open the door in response to measuring a force above a predefined threshold value. In some embodiments, the disinfection system can include a motor (or other drive system) for driving the door that has a clutch system (e.g., a magnetic clutch system). The clutch system can be configured to disengage the door from the motor when a force acting on the door is greater than a predefined threshold value. When a door safety issue is detected (3009: YES), then the disinfection system can present or communicate this to the user or an administrator, at 3016. In some embodiments, the disinfection system can communicate to the user the reason that the system is not safe or ready (e.g., that there is an object obstructing the door) and/or provide instructions to the user on how to make the disinfection system safe or ready for use.

In some embodiments, the disinfection system can include additional sensor(s) and/or other components for monitoring safety of the disinfection system. For example, the disinfection system can include one or more electrical sensors (e.g., a voltmeter, ammeter, etc.) that can detect whether an energy source (e.g., energy source 122) is detective. The disinfection system can include one or more scanning devices (e.g., cameras) that can determine whether the chamber has a hole or other defect.

When no safety issues are detected (e.g., 3007: NO, 3009: NO), then the disinfection system (e.g., as controlled by a processor such as, for example, processor 154, 2604) can close the door to the disinfecting area, at 3010. For example, the disinfection system can automatically, e.g., via a drive system such as a motor, close the door. While closing the door, the disinfection system can continue to monitor for any safety issues (e.g., motion and/or door safety issues), and if any safety issues are detected, the disinfection system can stop closing the door and/or re-open the door. In some embodiments, the disinfection system can receive an input from a user (e.g., via touchscreen or other I/O device), and in response to the input, close the door.

At 3012, the disinfection system can disinfect the object. Disinfecting the object can include one or more of activating the disinfection system (e.g., similar to 1404 as depicted and described with reference to FIG. 22), identifying a type of the object (e.g., similar to 1406 as depicted and described with reference to FIG. 22), identifying a disinfecting mode (e.g., similar to 1408 as depicted and described with reference to FIG. 22), and performing disinfection (e.g., similar to 1410 as depicted and described with reference to FIG. 22). Upon, during, or after performing the disinfection, the disinfection system can optionally log and report the disinfection, as described above with reference to method 2100 at 2112. In instances where the object includes an indicator tag, the disinfection system can optionally program the object indicator tag to show that the object is compliant with disinfection protocols, as described with reference to method 2100 at 2114. At 3016, the disinfection system can optionally present or communicate the status of the disinfection to the user, e.g., communicate using an onboard display that the disinfection is complete and/or send information regarding the disinfection to a remote device (e.g., via network 2620).

Those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims.

Also, various concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

As used herein, the terms "about" and/or "approximately" when used in conjunction with values and/or ranges generally refer to those values and/or ranges near to a recited value and/or range. In some instances, the terms "about" and "approximately" may mean within ± 10% of the recited value. The terms "about" and "approximately" may be used interchangeably.

## Claims

1. An apparatus, comprising:
a plurality of walls (212, 214, 216, 218) collectively defining a chamber (124) sized to receive an object, a set of walls from the plurality of walls (212, 214, 216, 218) each formed of a set of at least two modular units (212a, 212b, 212c, 212d) from a plurality of modular units, the plurality of walls (212, 214, 216, 218) including a back wall (21 8) and a set of side walls (212, 214);
a plurality of energy sources (222) disposed on the plurality of walls (212, 214, 216, 218), a set of energy sources from the plurality of energy sources (222) configured to provide energy having an intensity capable of disinfecting a surface of the object when the object has been received within the chamber (124);
a sensor (164) configured to read an indicator tag (2400, 2500) associated with the object; and
a processor (154) operatively coupled to the plurality of energy sources (222) and the sensor (164), the processor (154) configured to:
determine that the object needs to be disinfected based on information associated with the indicator tag (2400, 2500) obtained by the sensor (164);
in response to determining that the object needs to be disinfected based on the information associated with the indicator tag (2400, 2500), activate the plurality of energy sources (222) such that the plurality of energy sources (222) provides energy to disinfect the surface of the object; and
**characterised in that**
the plurality of walls (212, 214, 216, 218) include a top wall (216) and after disinfecting the surface of the object, the processor (154) is configured to send a signal receivable by the indicator tag (2400, 2500) such that the indicator tag (2400, 2500), in response to receiving the signal, can change a display of the indicator tag (2400, 2500) to reflect that the object has been disinfected.

2. The apparatus of claim 1, wherein the processor (154) is further configured to
after disinfecting the object, send information regarding the disinfection of the object receivable by a remote compute device (2660) such that the remote compute device (2660), in response to receiving the information regarding the disinfection, can update a log reflective of a disinfection history of the object.

3. The apparatus of claim 1, further comprising:
A control panel (150) including a user interface (152), the control panel (150) configured to present information to a user via the user interface (152) and to receive one or more inputs from the user.

4. The apparatus of claim 3, wherein the processor (154) is further configured to, in response to determining that the object does not need to be disinfected based on the information associated with the indicator tag (2400, 2500), cause the user interface (152) to display a message indicating that the object does not need to be disinfected.

5. The apparatus of any one of claims 3-4, wherein the processor (154) is configured to present, via the user interface (152), a set of instructions indicating steps to be performed by the user associated with the disinfection of the object.

6. The apparatus of any one of claims 3-5, wherein the control panel (150) includes a touchscreen.

7. The apparatus of any one of claims 3-6, wherein the sensor (164) is a first sensor, the apparatus further comprising:
a door configured to open to provide access to the chamber (124) and to close to seal the chamber (124),
the control panel (150) further including a second sensor configured to scan an identification badge of the user,
the processor (154) being operatively coupled to the door and the second sensor, the processor further configured to:
determine that the user is an authorized user based on information associated with the identification badge obtained by the second sensor; and
in response to determining that the user is an authorized user, automatically causing the door to open such that the object can be placed within the chamber (124).

8. The apparatus of 7, wherein the processor (154) is further configured to:
automatically cause the door to close after a predetermined period has elapsed between the door opening and the door closing and no object has been detected within the chamber (124).

9. The apparatus of claim 7, wherein the second sensor is configured to scan the identification badge a first time, the second sensor further configured to scan the identification badge a second time,
the processor (154) further configured to:
in response to detecting that the identification badge has been scanned a second time, automatically causing the door to close.

10. The apparatus of claim 7, wherein the processor (154) is further configured to:
after disinfecting the surface of the object, automatically cause the door to open such that the object can be removed from the chamber (124).

11. The apparatus of claim 1, wherein the processor (154) is further configured to:
identify a type of object based on the information associated with the indicator tag (2400, 2500); and
determine a type of disinfection based on the type of object,
the processor (154) configured to activate the plurality of energy sources (222) according to a set of protocols associated with the type of disinfection.

## Patentansprüche

1. Vorrichtung, umfassend:
eine Vielzahl von Wänden (212, 214, 216, 218), die gemeinsam eine Kammer (124) definieren, die so bemessen ist, dass sie ein Objekt aufnehmen kann, wobei ein Satz von Wänden aus der Vielzahl von Wänden (212, 214, 216, 218) jeweils aus einem Satz von mindestens zwei modularen Einheiten (212a, 212b, 212c, 212d) aus einer Vielzahl von modularen Einheiten gebildet ist, wobei die Vielzahl von Wänden (212, 214, 216, 218) eine Rückwand (218) und einen Satz von Seitenwänden (212, 214) umfasst;
eine Vielzahl von Energiequellen (222), die an der Vielzahl von Wänden (212, 214, 216, 218) angeordnet ist, wobei ein Satz von Energiequellen aus der Vielzahl von Energiequellen (222) so konfiguriert ist, dass er Energie mit einer Intensität bereitstellt, die in der Lage ist, eine Oberfläche des Objekts zu desinfizieren, wenn das Objekt in der Kammer (124) aufgenommen wurde;
einen Sensor (164), der so konfiguriert ist, dass er ein dem Objekt zugeordnetes Anzeigeetikett (2400, 2500) liest; und
einen Prozessor (154), der betriebsmäßig mit der Vielzahl von Energiequellen (222) und dem Sensor (164) gekoppelt ist, wobei der Prozessor (154) so konfiguriert ist, dass er:
auf Grundlage von dem Sensor (164) erhaltener, dem Anzeigeetikett (2400, 2500) zugeordneter Informationen bestimmt, dass das Objekt desinfiziert werden muss;
in Reaktion auf das auf Grundlage der dem Anzeigeetikett (2400, 2500) zugeordneten Informationen erfolgende Bestimmen, dass das Objekt desinfiziert werden muss, die Vielzahl von Energiequellen (222) aktiviert, so dass die Vielzahl von Energiequellen (222) Energie zum Desinfizieren der Oberfläche des Objekts bereitstellt; und
**dadurch gekennzeichnet, dass** die Vielzahl von Wänden (212, 214, 216, 218) eine obere Wand (216) umfasst und der Prozessor (154) so konfiguriert ist, dass er nach dem Desinfizieren der Oberfläche des Objekts ein Signal sendet, das von dem Anzeigeetikett (2400, 2500) empfangen werden kann, so dass das Anzeigeetikett (2400, 2500) in Reaktion auf das Empfangen des Signals eine Anzeige des Anzeigeetiketts (2400, 2500) ändern kann, um wiederzugeben, dass das Objekt desinfiziert wurde.

2. Vorrichtung nach Anspruch 1, wobei der Prozessor (154) ferner so konfiguriert ist, dass er:
nach dem Desinfizieren des Objekts Informationen bezüglich der Desinfektion des Objekts sendet, die von einer entfernten Rechenvorrichtung (2660) empfangen werden können, so dass die entfernte Rechenvorrichtung (2660) in Reaktion auf das Empfangen der Informationen bezüglich der Desinfektion ein Protokoll aktualisieren kann, das einen Desinfektionsverlauf des Objekts wiedergibt.

3. Vorrichtung nach Anspruch 1, ferner umfassend:
ein Bedienfeld (150) mit einer Benutzerschnittstelle (152), wobei das Bedienfeld (150) so konfiguriert ist, dass es einem Benutzer über die Benutzerschnittstelle (152) Informationen präsentiert und eine oder mehrere Eingaben von dem Benutzer empfängt.

4. Vorrichtung nach Anspruch 3, wobei der Prozessor (154) ferner so konfiguriert ist, dass er in Reaktion auf ein auf Grundlage der dem Anzeigeetikett (2400, 2500) zugeordneten Informationen erfolgendes Bestimmen, dass das Objekt nicht desinfiziert werden muss, die Benutzerschnittstelle (152) veranlasst, eine Nachricht anzuzeigen, die angibt, dass das Objekt nicht desinfiziert werden muss.

5. Vorrichtung nach einem der Ansprüche 3 und 4, wobei der Prozessor (154) so konfiguriert ist, dass er über die Benutzerschnittstelle (152) einen Satz von Anweisungen präsentiert, die vom Benutzer auszuführende Schritte im Zusammenhang mit der Desinfektion des Objekts angeben.

6. Vorrichtung nach einem der Ansprüche 3 bis 5, wobei das Bedienfeld (150) einen Touchscreen umfasst.

7. Vorrichtung nach einem der Ansprüche 3 bis 6, wobei der Sensor (164) ein erster Sensor ist und die Vorrichtung ferner umfasst:
eine Tür, die so konfiguriert ist, dass sie sich öffnet, um Zugang zu der Kammer (124) zu gewähren, und dass sie sich schließt, um die Kammer (124) zu verschließen,
wobei das Bedienfeld (150) ferner einen zweiten Sensor umfasst, der so konfiguriert ist, dass er einen Identifikationsausweis des Benutzers scannt,
wobei der Prozessor (154) betriebsmäßig mit der Tür und dem zweiten Sensor gekoppelt ist, wobei der Prozessor ferner so konfiguriert ist, dass er:
auf Grundlage durch den zweiten Sensor erhaltener Informationen im Zusammenhang mit dem Identifikationsausweis bestimmt, dass es sich bei dem Benutzer um einen autorisierten Benutzer handelt; und
in Reaktion auf das Bestimmen, dass es sich bei dem Benutzer um einen autorisierten Benutzer handelt, automatisch ein Öffnen der Tür veranlasst, so dass das Objekt in der Kammer (124) platziert werden kann.

8. Vorrichtung nach Anspruch 7, wobei der Prozessor (154) ferner so konfiguriert ist, dass er:
automatisch veranlasst, dass sich die Tür schließt, nachdem eine vorbestimmte Zeitspanne zwischen dem Öffnen der Tür und dem Schließen der Tür verstrichen ist und kein Objekt in der Kammer (124) erkannt wurde.

9. Vorrichtung nach Anspruch 7, wobei der zweite Sensor so konfiguriert ist, dass er den Identifikationsausweis ein erstes Mal scannt, wobei der zweite Sensor ferner so konfiguriert ist, dass er den Identifikationsausweis ein zweites Mal scannt,
wobei der Prozessor (154) ferner so konfiguriert ist, dass er:
in Reaktion auf Erkennen, dass der Identifikationsausweis ein zweites Mal gescannt wurde, automatisch veranlasst, dass sich die Tür schließt.

10. Vorrichtung nach Anspruch 7, wobei der Prozessor (154) ferner so konfiguriert ist, dass er:
nach dem Desinfizieren der Oberfläche des Objekts automatisch veranlasst, dass sich die Tür öffnet, so dass das Objekt aus der Kammer (124) entnommen werden kann.

11. Vorrichtung nach Anspruch 1, wobei der Prozessor (154) ferner so konfiguriert ist, dass er:
auf Grundlage der dem Anzeigeetikett (2400, 2500) zugeordneten Informationen einen Objekttyp identifizert; und
auf Grundlage des Objekttyps einen Desinfektionstyp bestimmt,
wobei der Prozessor (154) so konfiguriert ist, dass er die Vielzahl von Energiequellen (222) gemäß einem dem Desinfektionstyp zugeordneten Satz von Protokollen aktiviert.

## Revendications

1. Appareil, comprenant :
une pluralité de parois (212, 214, 216, 218) définissant collectivement une chambre (124) dimensionnée de manière à recevoir un objet, un ensemble de parois parmi la pluralité de parois (212, 214, 216, 218) étant chacune formées d'un ensemble d'au moins deux unités modulaires (212a, 212b, 212c, 212d) parmi une pluralité d'unités modulaires, la pluralité de parois (212, 214, 216, 218) comportant une paroi arrière (218) et un ensemble de parois latérales (212, 214) ;
une pluralité de sources d'énergie (222) placées sur la pluralité de parois (212, 214, 216, 218), un ensemble de sources d'énergie parmi la pluralité de sources d'énergie (222) étant configurées pour fournir de l'énergie dont l'intensité est apte à désinfecter une surface de l'objet lorsque l'objet a été reçu à l'intérieur de la chambre (124) ;
un capteur (164) configuré pour lire une étiquette indicatrice (2400, 2500) associée à l'objet ; et
un processeur (154) couplé fonctionnellement à la pluralité de sources d'énergie (222) et au capteur (164), le processeur (154) étant configuré pour :
déterminer que l'objet a besoin d'être désinfecté sur la base d'informations associées à l'étiquette indicatrice (2400, 2500) obtenues par le capteur (164) ;
en réponse à la détermination que l'objet a besoin d'être désinfecté sur la base des informations associées à l'étiquette indicatrice (2400, 2500), activer la pluralité de sources d'énergie (222) de façon à ce que la pluralité de sources d'énergie (222) fournissent de l'énergie pour désinfecter la surface de l'objet ; et
**caractérisé en ce que** la pluralité de parois (212, 214, 216, 218) comportent une paroi de dessus (216) et, après la désinfection de la surface de l'objet, le processeur (154) est configuré pour envoyer un signal susceptible d'être reçu par l'étiquette indicatrice (2400, 2500) de façon à permettre à l'étiquette indicatrice (2400, 2500), en réponse à la réception du signal, de modifier un affichage de l'étiquette indicatrice (2400, 2500) pour refléter que l'objet a été désinfecté.

2. Appareil selon la revendication 1, le processeur (154) étant configuré en outre pour :
après la désinfection de l'objet, envoyer des informations concernant la désinfection de l'objet susceptibles d'être reçues par un dispositif informatique (2660) distant de façon à permettre au dispositif informatique (2660) distant, en réponse à la réception des informations concernant la désinfection, de mettre à jour un journal reflétant un historique de désinfections de l'objet.

3. Appareil selon la revendication 1, comprenant en outre :
un panneau de commande (150) comportant une interface utilisateur (152), le panneau de commande (150) étant configuré pour présenter des informations à un utilisateur via l'interface utilisateur (152) et pour recevoir une ou plusieurs entrées émanant de l'utilisateur.

4. Appareil selon la revendication 3, le processeur (154) étant configuré en outre pour, en réponse à la détermination que l'objet n'a pas besoin d'être désinfecté sur la base des informations associées à l'étiquette indicatrice (2400, 2500), amener l'interface utilisateur (152) à afficher un message indiquant que l'objet n'a pas besoin d'être désinfecté.

5. Appareil selon l'une quelconque des revendications 3 à 4, le processeur (154) étant configuré pour présenter, via l'interface utilisateur (152), un ensemble d'instructions indiquant des étapes à réaliser par l'utilisateur associées à la désinfection de l'objet.

6. Appareil selon l'une quelconque des revendications 3 à 5, le panneau de commande (150) comportant un écran tactile.

7. Appareil selon l'une quelconque des revendications 3 à 6, le capteur (164) étant un premier capteur, l'appareil comprenant en outre :
une porte configurée pour s'ouvrir pour donner accès à la chambre (124) et pour se fermer pour fermer la chambre (124) de manière étanche,
le panneau de commande (150) comportant en outre un deuxième capteur configuré pour scanner un badge d'identification de l'utilisateur,
le processeur (154) étant couplé fonctionnellement à la porte et au deuxième capteur, le processeur étant configuré en outre pour :
déterminer que l'utilisateur est un utilisateur autorisé sur la base d'informations associées au badge d'identification obtenues par le deuxième capteur ; et
en réponse à la détermination que l'utilisateur est un utilisateur autorisé, commander l'ouverture automatique de la porte de façon à permettre de placer l'objet à l'intérieur de la chambre (124).

8. Appareil selon la revendication 7, le processeur (154) étant configuré en outre pour :
commander la fermeture automatique de la porte après qu'un laps de temps prédéterminé s'est écoulé entre l'ouverture de la porte et la fermeture de la porte et qu'aucun objet n'a été détecté à l'intérieur de la chambre (124).

9. Appareil selon la revendication 7, le deuxième capteur étant configuré pour scanner le badge d'identification une première fois, le deuxième capteur étant configuré en outre pour scanner le badge d'identification une deuxième fois,
le processeur (154) étant configuré en outre pour :
en réponse à la détection que le badge d'identification a été scanné une deuxième fois, commander la fermeture automatique de la porte.

10. Appareil selon la revendication 7, le processeur (154) étant configuré en outre pour :
après la désinfection de la surface de l'objet, commander l'ouverture automatique de la porte de façon à permettre de retirer l'objet de la chambre (124).

11. Appareil selon la revendication 1, le processeur (154) étant configuré en outre pour :
identifier un type d'objet sur la base des informations associées à l'étiquette indicatrice (2400, 2500) ; et
déterminer un type de désinfection sur la base du type d'objet,
le processeur (154) étant configuré pour activer la pluralité de sources d'énergie (222) selon un ensemble de protocoles associés au type de désinfection.
